(19) Europäisches Patentamt
European Patent Office
Office européen des brevets

(11) **EP 3 737 756 B1**

(12) **EUROPEAN PATENT SPECIFICATION**

(45) Date of publication and mention of the grant of the patent:
**08.12.2021 Bulletin 2021/49**

(51) Int Cl.:
*C12N 15/10* $^{(2006.01)}$    *C12N 15/63* $^{(2006.01)}$
*C12Q 1/6806* $^{(2018.01)}$    *G16B 15/10* $^{(2019.01)}$

(21) Application number: **19714818.2**

(22) Date of filing: **07.01.2019**

(86) International application number:
**PCT/NL2019/050006**

(87) International publication number:
**WO 2019/139473 (18.07.2019 Gazette 2019/29)**

(54) **METHODS AND SYSTEMS FOR SUPERCOILING DNA**

VERFAHREN UND SYSTEME ZUM SUPERCOILING VON DNA

MÉTHODES ET SYSTÈMES DE SUPERENROULEMENT D'ADN

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **09.01.2018 NL 2020257**

(43) Date of publication of application:
**18.11.2020 Bulletin 2020/47**

(73) Proprietor: **LUMICKS DSM Holding B.V.**
**1059 CH Amsterdam (NL)**

(72) Inventors:
• **KING, Graeme Alan**
**1081 HV Amsterdam (NL)**
• **BURLA, Federica**
**1081 HV Amsterdam (NL)**
• **WUITE, Gijs Jan Lodewijk**
**1081 HV Amsterdam (NL)**
• **PETERMAN, Erwin Johannes Gerard**
**1081 HV Amsterdam (NL)**

(74) Representative: **De Vries & Metman**
**Overschiestraat 180**
**1062 XK Amsterdam (NL)**

(56) References cited:
• KING GRAEME A ET AL: "Unravelling the structural plasticity of stretched DNA under torsional constraint.", NATURE COMMUNICATIONS, vol. 7, no. 11810, 6 June 2016 (2016-06-06), pages 1-7, XP002783280, ISSN: 2041-1723 cited in the application

• KING GRAEME A ET AL: "Supplementary Information: Unravelling the structural plasticity of stretched DNA under torsional constraint.", NATURE COMMUNICATIONS 06 JUN 2016, 6 June 2016 (2016-06-06), XP002783281, ISSN: 2041-1723, DOI: 10.1038/ncomms11810 Retrieved from the Internet: URL:https://www.nature.com/articles/ncomms 11810#supplementary-information [retrieved on 2018-07-23] cited in the application
• VAN MAMEREN JOOST ET AL: "Unraveling the structure of DNA during overstretching by using multicolor, single-molecule fluorescence imaging.", PROCEEDINGS OF THE NATIONAL ACADEMY OF SCIENCES OF THE UNITED STATES OF AMERICA, vol. 106, no. 43, 27 October 2009 (2009-10-27), pages 18231-18236, XP002783282, ISSN: 1091-6490 cited in the application
• J.F.LÉGER ET AL.: "Structural Transitions of a twisted and stretched DNA molecule", PHYSICAL REVIEW LETTERS, vol. 83, no. 5, 2 August 1999 (1999-08-02), pages 1066-1069, XP002783283, The American Physical Society, US cited in the application
• QIAN WANG ROSSITZA N ET AL: "Influence of DNA sequence on the structure of minicircles under torsional stress", 20170727, vol. 45, no. 13, 27 July 2017 (2017-07-27), pages 7633-7642, XP002782272, ISSN: 0305-1048, DOI: 10.1093/NAR/GKX516 cited in the application

EP 3 737 756 B1

**(Cont. next page)**

- KING GRAEME A ET AL: "Revealing the competition between peeled ssDNA, melting bubbles, and S-DNA during DNA overstretching using fluorescence microscopy.", PROCEEDINGS OF THE NATIONAL ACADEMY OF SCIENCES OF THE UNITED STATES OF AMERICA, vol. 110, no. 10, 5 March 2013 (2013-03-05), pages 3859-3864, XP002783284, ISSN: 1091-6490
- VAN LOENHOUT M T J ET AL: "Dynamics of DNA supercoils.", SCIENCE, vol. 338, no. 6103, 5 October 2012 (2012-10-05), pages 94-97, XP002783285, NEW YORK, N.Y ISSN: 1095-9203 cited in the application
- PINCET FRÉDÉRIC ET AL: "The solution to the streptavidin-biotin paradox: the influence of history on the strength of single molecular bonds.", BIOPHYSICAL JOURNAL, vol. 89, no. 6, December 2005 (2005-12), pages 4374-4381, XP002783286, ISSN: 0006-3495 cited in the application
- KING GRAEME A. AND WUITE GIJS J.L.: "Biophysical chemistry: Strength in numbers.", NATURE CHEMISTRY, vol. 6, no. 1, January 2014 (2014-01), pages 13-14, XP002783287, ISSN: 1755-4349
- CHAURASIYA KATHY R ET AL: "Oligomerization transforms human APOBEC3G from an efficient enzyme to a slowly dissociating nucleic acid-binding protein.", NATURE CHEMISTRY, vol. 6, no. 1, January 2014 (2014-01), pages 28-33, XP002783288, ISSN: 1755-4349
- VAN MAMEREN JOOST ET AL: "A polarized view on DNA under tension.", THE JOURNAL OF CHEMICAL PHYSICS ONLINE, vol. 148, no. 12, 11 December 2017 (2017-12-11), pages 1-9, XP002790797, ISSN: 1089-7690, DOI: 10.1063/1.5004019
- SCHAKENRAAD KOEN ET AL: "Hyperstretching DNA", NATURE COMMUNICATIONS, vol. 8, 19 December 2017 (2017-12-19), XP085342417, ISSN: 2041-1723
- SCHLINGMAN DANIEL J ET AL: "A new method for the covalent attachment of DNA to a surface for single-molecule studies.", COLLOIDS AND SURFACES. B, BIOINTERFACES, vol. 83, no. 1, March 2011 (2011-03), pages 91-95, XP027562737, ISSN: 1873-4367 cited in the application
- KING GRAEME A ET AL: "Direct Observation and Quantification of Protein Dynamics on Negatively-Supercoiled DNA, Abstract No. 847 Pos Board B617;", BIOPHYSICAL JOURNAL, vol. 114, no. 3, Suppl. 1, 2 February 2018 (2018-02-02), page 168A, XP002790798, & 62ND ANNUAL MEETING OF THE BIOPHYSICAL-SOCIETY; SAN FRANCISCO, CA, USA; FEBRUARY 17 -21, 2018 ISSN: 0006-3495

**Description**

**FIELD OF THE INVENTION**

This disclosure relates to methods and systems for supercoiling DNA.

**BACKGROUND**

Cellular DNA is regularly subject to torsional stress during genomic processes, resulting in changes to the topological state of DNA. The topological state of DNA is described by the linking number (Lk), which is the number of times one of the strands of the double-stranded DNA molecule crosses the other strand. The linking number is a linear combination of the twist (*Tw*) and writhe (Wr) of the DNA:

$$Lk = Tw + Wr \quad (Eq. 1)$$

An applied torque can induce changes in twist, which corresponds to either underwinding or overwinding of the double-helix, or changes in writhe, resulting in either the formation or loss of buckled structures in which disparate regions of the DNA cross one another, e.g. plectonemes. If the linking number of DNA is altered relative to its native form, the DNA is said to be supercoiled.

Since the double-helical backbone of DNA is chiral, supercoiling can be either positive or negative, depending on the direction of the applied torque. Torque that is acting in the same direction as the handedness of the backbone of the double-helix can induce positive supercoiling, corresponding to either positive twist (overwinding) or positive writhe (e.g. positive plectonemes). Conversely, torque that is acting in the opposite direction to the handedness of the backbone of the double-helix can induce negative supercoiling, corresponding to either negative twist (underwinding) or negative writhe (e.g. negative plectonemes).

The extent of supercoiling ($\sigma$) is typically defined as the change in linking number (*Lk*), relative to a starting linking number ($Lk_0$):

$$\sigma = (Lk - Lk_0) / Lk_0 \quad (Eq. 2)$$

In vivo, both negatively-supercoiled and positively-supercoiled DNA structures are frequently generated, and, overall, cellular DNA is typically slightly negatively-supercoiled. In vivo, $\sigma$ is generally thought to be between ~-0.1 and 0. Additionally, the study of supercoiled DNA is important for gaining insight into the mechanistic basis for many diseases, as well as for investigating the influence of certain drugs on processes that involve supercoiled DNA.

Thus, the ability to sensitively detect and analyze the biophysical properties of supercoiled DNA, as well as the interactions of entities, such as proteins, dyes and drugs, with supercoiled DNA, is of great importance as a means to understand a wide range of genomic processes. Of course, the ability to supercoil DNA is very important for such experiments.

A method for supercoiling DNA is known from Van Loenhout, M.T.J. Science, 338, 94 (2012), which involves magnetic tweezers. Herein a DNA molecule is tethered between a magnetic bead and a fixed surface. Then, by rotating a magnet, torque can be applied to the DNA molecule via the magnetic bead.

Disadvantageously, achieving high degrees of supercoiling with this method is time consuming, because the magnetic beads must be controlled to rotate numerous times. This is especially true when long DNA molecules are to be supercoiled. To illustrate, a rough estimate is that one turn of the magnet typically takes ~0.3 seconds. For lambda-phage DNA, which has a ~48500 base-pairs length, approximately 3000 turns of the magnet would be required to generate a high (sigma ~ -0.7) extent of supercoiling. Therefore, it could take up to ~1000 seconds to supercoil the molecule in this case.

Another disadvantage of the method involving magnetic tweezers is that it does not allow easy control of the orientation and/or position of the DNA molecule, because it is at one end physically tethered to an immovable surface. As a result, solution conditions cannot be exchanged rapidly, which decreases the ability to measure and/or image ligand-binding to supercoiled DNA in a ligand-free solution. Further, the DNA molecule cannot be easily oriented perpendicular to an optical axis of an objective of a microscope, i.e. oriented "horizontally" in the focal plane of the objective. Therefore, it can be challenging to align the DNA molecule in a horizontal orientation, which makes it technically challenging to combine this method with fluorescence microscopy. Tilting the magnet, and thus the DNA molecule, at an angle to a fixed surface still does not satisfactorily achieve that the DNA molecule is kept horizontal and can also cause unwanted surface interactions between the DNA and the surface. Orienting the DNA molecule by tilting the magnet also requires significant technical expertise. Furthermore, since the tether point between the DNA and the magnetic bead typically does not coincide with the axis of rotation of the bead induced by the magnetic moment, the DNA molecule is typically not attached to the point on the magnetic bead which is closest to the other tether point which complicates determination of the true end-to-end distance of the DNA molecule.

Hence, there is a need in the art for a method for supercoiling a DNA molecule that alleviates at least some of the above-identified problems.

## SUMMARY

The invention is defined by the scope of the appended claims.

A method for supercoiling DNA, e.g. negatively supercoiling DNA, is disclosed. In an initial state, at least part of a DNA molecule is torsionally constrained and associated with a first linking number. The at least part of the DNA molecule has a first end connected to a first body and a second end connected to a second body. The method comprises increasing a distance between the first body and the second body for inducing a torque in the at least part of the DNA molecule and for bringing the at least part of the DNA molecule from the initial state into an intermediate state. In the intermediate state the at least part of the DNA molecule is temporarily torsionally unconstrained for at least partially releasing the induced torque for changing, e.g. decreasing, the first linking number. The method further comprises decreasing the distance between the first and second body for bringing the at least part of the DNA molecule from the intermediate state into a further state in which the at least part of the DNA molecule is torsionally constrained and associated with a second linking number different from the first linking number.

Preferably, decreasing the distance starts when the at least part of the DNA molecule in the intermediate state is torsionally constrained again. Lowering the tension on the DNA molecule when it is torsionally unconstrained (by decreasing the distance between the first and second body), may namely reduce the degree of supercoiling that was achieved at the maximum distance between the first and second body.

A torsional constraint on the at least part of the DNA molecule may thus be re-established when the DNA molecule is in the intermediate state. Subsequently decreasing the distance between the first and second body may be understood to stabilize this torsional constraint. Preferably, the torsional constraint that is stabilized due to the distance decrease is present when the distance starts to decrease.

When the at least part of the DNA molecule is said to be torsionally constrained, it may be understood as that the overall linking number of the at least part of the DNA molecule cannot change.

Since the DNA molecule acts as a spring, increasing the distance between the first and second body may be understood as equivalent to applying a tension to the DNA molecule.

Hence, the method advantageously enables to create supercoiled DNA, that remains supercoiled at low tensions, relatively fast by using the intrinsic mechanical properties of DNA, in particular the property of DNA that it wants to unwind as it is extended. The method does not require actively rotating either one of the first or second body.

Furthermore, the method does not require the DNA molecule to be attached to any fixed structure, such as a flat immovable surface or a micropipette. Therefore, the supercoiled DNA molecule can be easily oriented and positioned, especially if the first and second body are optically trapped beads. The method can thus be easily combined with for example fluorescence microscopy. The method in particular allows to combine a dual tweezer setup with supercoiled DNA.

In one embodiment, the first end of the at least part of the DNA molecule is connected by at least two bonds to the first body when the at least part of the DNA molecule is torsionally constrained. Preferably, the second end is also connected by at least two bonds to the second body when the at least part of the DNA molecule is torsionally constrained.

The bonds may be non-covalent bonds, such as streptavidin-biotin bonds.

Increasing the distance between the first and second body may be understood to destabilize at least one of, but possibly all of, the bonds. Destabilizing a bond may be understood as increasing the dissociation constant of this bond and/or as increasing the probability that the bond dissociates during a time period. Stabilizing a bond may be understood as decreasing the dissociation constant of the bond and/or as decreasing the probability that the bond dissociates during a time period.

The dissociation constant of a bond typically depends on the force that is applied to it. A higher applied force is associated with a higher dissociation constant. Hence, at high applied forces, dissociation of such a bond is more likely to occur and thus is expected to occur more frequently.

In particular, bonds between the DNA and one or both of the two bodies are believed to possess a stability that depends not only on the tension applied to the DNA molecule, but probably also on the torque that the DNA molecule applies to the bond. The torque may namely function as a catalyst for rupturing the at least one bond.

When, at the first end of the at least part of the DNA molecule, all but one of the bonds are dissociated, the DNA molecule is torsionally unconstrained as it can freely rotate around the remaining bond. Herewith, the induced torque is at least partially released and the linking number of the DNA molecule changes. Of course, when all bonds break the DNA molecule is no longer connected to the first and/or second body.

A possible explanation for the preservation of the one remaining bond, despite this bond having to endure the force applied to the DNA molecule on its own, is that the release of the torsional stress prevents rupture of the bond. An additional or alternative explanation is that the at least two bonds have different strengths.

It should be appreciated that after all but one of the bonds between the DNA and the first body have dissociated, bonds may be formed again, which may be the result of a stochastic binding process. Also, the reduced torque on the DNA molecule may increase the probability that a bond is formed or reformed. Once the DNA molecule is connected again by at least two bonds to the first body and preferably also by at least two bonds to the second body, the DNA molecule is torsionally constrained again. The molecule can thus resume a torsionally constrained state at relatively high tension, but with a lower linking number than prior to increasing the distance between the first and second body. In light of the foregoing, decreasing the distance between the first and second body may be understood to stabilize the bonds. Thus, decreasing the distance may be understood to stabilize the newly applied torsional constraint on the at least part of the DNA molecule. Hence, the DNA molecule remains torsionally constrained at low tensions. The DNA molecule thus cannot revert to its initial state, wherein it was not supercoiled.

Preferably, the distance between the two bodies starts to decrease when the DNA molecule is connected by means of at least two bonds to the first body such that the DNA molecule is torsionally constrained. Preferably, the distance between the two bodies starts to decrease when the DNA molecule is connected by means of at least two bonds to the second body as well. For example, the distance starts to decrease before one of the bonds dissociates again. Lowering the tension on the DNA molecule when it can freely rotate around a single bond to the first body, may namely reduce the degree of supercoiling that was achieved at the maximum distance between the first and second body.

In this embodiment, the DNA molecule is torsionally constrained in a convenient manner.

In one embodiment, the at least two bonds between the first end of the at least part of the DNA molecule and the first body comprise a first bond and at least one other bond, the at least one other bond being a non-covalent bond, preferably a non-covalent biotin-type bond. Preferably, the second end is connected to the second body by means of at least two bonds, a third bond and at least one other bond, the at least one other bond being a non-covalent bond, preferably a non-covalent biotin-type bond. Preferably, all bonds, including the first and third bond, between the DNA molecule and the two bodies are of the same type. Alternatively, said first bond and/or said third bond may be a covalent bond as described in Colloids Surf B Biointerfaces, 2011 March, 83(1): 91-95.

In one embodiment, the above mentioned at least one other bond at the first end of the DNA molecule is a streptavidin-biotin bond. Preferably, the above mentioned at least one other bond at the second end of the DNA molecule is also a streptavidin-biotin bond. More preferably, all bonds, including the first, and optionally third, bond, between the DNA molecule and the two bodies are streptavidin-biotin bonds.

The at least one other bond at either end may also be an avidin-biotin bond, a traptavidin-biotin bond and/or a NeutrAvidin-biotin bond.

In particular, streptavidin-biotin bonds are sufficiently stable at high forces, which allows to apply relatively high tensions to the DNA molecule, e.g. tensions larger than 60 pN, preferably larger than 100 pN, as a result of which tensions the DNA molecule prefers to be in a supercoiled state (e.g. comprising a lower linking number) when not torsionally constrained. At the same time, the streptavidin-biotin bond is sufficiently unstable so that at high forces, at least one of them can rupture so that only one such bond remains for releasing the induced torque on the DNA molecule. Hence, streptavidin-biotin is particularly suitable for the methods described herein.

Thus, in one embodiment, when the at least part of the DNA molecule is torsionally constrained, the first end is connected by at least two, for example two, three, four or five, streptavidin-biotin bonds to the first body and the second end is connected by at least two streptavidin-biotin bonds to the second body.

One embodiment comprises controlling the position of the first body relative to the second body using a trapping system, preferably an optical trapping system or an acoustical trapping system.

The first and/or second body may sit in a trap, such as an optical or acoustical trap. Changing the distance between the first and second body may thus comprise controlling a trapping system, such as an optical trapping system, to move said traps with respect to each other.

Further, increasing the distance between the first and second body may include using the trapping system to control the position of the first and second body in order to increase the distance.

This embodiment enables to easily manipulate the distance between the two bodies. Suspending the bodies in two traps yields several important advantages. One advantage is that the suspended DNA molecule is decoupled from the sample holder which can prevent mechanical drift from coupling to the manipulation system. Another advantage is that rapid fluid exchange is possible. In a (laminar flow) microfluidic system the DNA molecule can be quickly moved into a different fluid channel by moving the sample stage and/or the traps. A further advantage is that the DNA molecule can be easily aligned with a measurement scan line by manipulating the optical traps, which enables for fast optical interrogation of the DNA molecule. A focus may namely be scanned over the DNA in one dimension, which requires alignment of the molecule to the scan line.

In one embodiment, the distance increase causes a tension to be applied to the DNA molecule that exceeds 60 pN, preferably 80 pN, more preferably 100 pN, most preferably 115 pN. At sufficient applied force, the DNA molecule overstretches, which is beneficial because it induces a substantial torque in the at least part of the DNA molecule. If the first and/or second body sits in an optical trap, the force exerted by a trap on a body may be measured using e.g. video tracking or back-focal plane interferometry known in the art.

In one embodiment, decreasing the distance reduces a tension in the DNA molecule below 100 pN, preferably below 80 pN, more preferably below 50 pN, most preferably below 20 pN or 10 pN. This embodiment thus allows supercoiled DNA to be prepared and/or studied at forces lower than those associated with overstretching - which is relevant for many biophysical studies.

In one embodiment, providing the DNA molecule comprises providing the first body comprising streptavidin for forming a bond with a biotin molecule, connecting at least one biotin molecule to the DNA molecule and combining the first body and the DNA molecule in a fluid for causing a bond to form between said streptavidin of the first body and the at least one biotin molecule connected to the DNA molecule. The first body may comprise streptavidin at its surface. In particular, the first body may be at least partially coated with streptavidin. This embodiment provides a convenient manner for providing the DNA molecule.

In one embodiment, the DNA molecule comprises at its first and/or second end an end-cap structure. This embodiment optionally comprises attaching an end-cap structure to at least one end of the DNA molecule. The DNA molecule thus may have a closed-end structure, which may be referred to as a loop end or a hairpin structure. The end-cap structure optionally comprises at least one biotin molecule for forming a bond with streptavidin.

In one embodiment, when the DNA molecule is torsionally constrained, two free backbone strands of the DNA molecule at the first end of the DNA molecule are each connected to the first body, and optionally two free backbone strands of the DNA molecule at the second end of the DNA molecule are each connected to the second body. This embodiment optionally comprises attaching at one end of the DNA molecule at least one biotin molecule to a free backbone strand of the DNA molecule for connecting the strand to the first body. This embodiment may comprise connecting at least one biotin molecule to a plurality, e.g. all four, of the free strands of the DNA molecule respectively.

In one embodiment, the method comprises obtaining reference information relating combinations of applied tension to DNA and extension of DNA to respective degrees of supercoiling of DNA. This embodiment further comprises, based on a particular combination of tension on the DNA molecule and extension of the DNA molecule, e.g. occurring during decreasing the distance between the first and second body, and based on the reference information, determining the degree of supercoiling of the DNA molecule. Obtaining reference information may comprise receiving and/or storing the reference information in a data processing system. This embodiment enables to determine the degree of supercoiling achieved using methods as described herein.

In one embodiment, based on a desired degree of supercoiling, the method comprises determining a maximum force to be applied to the at least part of the DNA molecule and increasing the distance between the first and second body until the maximum force is applied to the DNA molecule. This embodiment advantageously enables to, at least to some extent, control the degree of supercoiling because the degree of supercoiling depends on the maximum force applied to the DNA molecule.

In one embodiment, the method comprises, based on a desired degree of supercoiling, determining at what point in time to start decreasing the distance and decreasing the distance at said point in time. This embodiment advantageously allows to control the degree of supercoiling because the degree of supercoiling depends on how long the DNA molecule is kept under tension. The longer it is kept under tension, the longer the DNA molecule will be in a torsionally unconstrained state for releasing induced torque and the more the linking number will change. Thus, by controlling the force and/or the time spent at high force the extent of supercoiling can be controlled.

After increasing the distance between the first and the second body, the distance and/or tension may be maintained substantially constant for a time period ranging typically from around 0.5 seconds up to many minutes.

This embodiment provides a convenient way for controlling the total time during which the at least one streptavidin-biotin bond is dissociated while the DNA molecule is under tension. The bond may rupture and reform numerous times while a tension is applied to the DNA. The longer the molecule is kept under tension, the more chance it is given to partially unwind. Even if the periods in which the at least one streptavidin-biotin bond is continuously dissociated only last for very short time durations, by keeping the DNA at tension long enough, it gives it the chance to unwind partially numerous times, such that the DNA molecule can revert to its equilibrium state given the specific tension that is applied, which state is of course a supercoiled state.

In one embodiment, the method comprises controlling at least one of a temperature of a fluid, a composition of a fluid and a light source for controlling a stability of the torsional constraint on the at least part of the DNA molecule, for example for controlling the stability of the at least one other bond mentioned above. The DNA molecule, first and second body, and in particular the bonds between these entities may be positioned in the fluid. The composition of the fluid may be controlled in the sense that at least one of an ionic strength of the fluid, a viscosity of the fluid and a pH of the fluid is controlled. This embodiment provides a convenient way for controlling the stability of the torsional constraint and thus a convenient manner for controlling the degree of supercoiling achieved. To illustrate, if the dissociation constant of the at least one other bond mentioned above increases with an increased temperature, increasing the temperature of the fluid will thus increase the dissociation constant. Consequently the stability of the torsional constraint decreases, since it becomes more likely that the at least one other bond dissociates leaving only the first bond intact, which by itself cannot torsionally constrain the at least part of the DNA molecule.

In one embodiment, the DNA molecule in the initial state has a first, optionally end-capped, end connected to the first body by means of at least two streptavidin-biotin bonds and a second, optionally end-capped, end connected by means of at least two streptavidin-biotin bonds to a second body. In this embodiment, the method comprises increasing the distance between the first body and the second body for inducing a torque in the DNA molecule and for destabilizing the streptavidin-biotin bonds for bringing the DNA molecule from the initial state into the intermediate state, in which temporarily at the first and/or second end all but one of the streptavidin-biotin bonds are dissociated for at least partially releasing the induced torque for changing, e.g. decreasing, the first linking number. In this embodiment, the method also comprises decreasing the distance between the first and second body for bringing the at least part of the DNA molecule from the intermediate state into the further state in which the first end is connected to the first body by means of at least two streptavidin-biotin bonds and the second end connected by means of at least two streptavidin-biotin bonds to a second body.

One aspect of this disclosure relates to a system for supercoiling DNA. The system comprises a trapping system for establishing at least one trap, e.g. an optical trap, for trapping at least one of a first and second body. At least part of a DNA molecule has a first end connected to the first body and a second end connected to the second body. The system further comprises a control module for controlling the trapping system for controlling a position of the at least one trap. The control module comprises a computer readable storage medium having computer readable program code embodied therewith, and a processor, preferably a microprocessor, coupled to the computer readable storage medium, wherein responsive to executing the computer readable program code, the processor is configured to perform executable operations comprising:

controlling the position of the at least one trap for increasing a distance between the first body and the second body for inducing a torque in the at least part of the DNA molecule and for bringing the at least part of the DNA molecule from the initial state into an intermediate state in which the at least part of the DNA molecule is temporarily torsionally unconstrained for at least partially releasing the induced torque for changing, e.g. decreasing, the first linking number, and

controlling the position of the at least one trap for decreasing the distance between the first and second body for bringing the at least part of the DNA molecule from the intermediate state into a further state in which the at least part of the DNA molecule is torsionally constrained and associated with a second linking number different from the first linking number.

The control module may be configured to perform any of the method steps as described herein. To illustrate, in one embodiment, the processor of the control module is configured to perform executable operations comprising obtaining reference information relating combinations of applied tension to DNA and extension of DNA to respective degrees of supercoiling of DNA and, based on a particular combination of tension on the DNA molecule and extension of the DNA molecule and, based on the reference information, determining a degree of supercoiling of the DNA molecule.

One aspect of this disclosure relates to a computer comprising a computer readable storage medium having computer readable program code embodied therewith, and a processor, preferably a microprocessor, coupled to the computer readable storage medium, wherein responsive to executing the computer readable program code, the processor is configured to perform one or more of the method steps as described herein.

One aspect of this disclosure relates to a computer program or suite of computer programs comprising at least one software code portion or a computer program product storing at least one software code portion, the software code portion, when run on a computer system, being configured for executing one or more of the method steps as described herein.

One aspect of this disclosure relates to a non-transitory computer-readable storage medium storing at least one software code portion, the software code portion, when executed or processed by a computer, is configured to perform one or more of the method steps as described herein.

As will be appreciated by one skilled in the art, aspects of the present disclosure may be embodied as a system, a method or a computer program product. Accordingly, aspects of the present disclosure may take the form of an entirely hardware embodiment, an entirely software embodiment (including firmware, resident software, micro-code, etc.) or an embodiment combining software and hardware aspects that may all generally be referred to herein as a "circuit," "module" or "system." Functions described in this disclosure may be implemented as an algorithm executed by a processor/microprocessor of a computer. Furthermore, aspects of the present disclosure may take the form of a computer program product embodied in one or more computer readable medium(s) having computer readable program code embodied, e.g., stored, thereon.

Any combination of one or more computer readable medium(s) may be utilized. The computer readable medium may be a computer readable signal medium or a computer readable storage medium. A computer readable storage medium may be, for example, but not limited to, an electronic, magnetic, optical, electromagnetic, infrared, or semiconductor system, apparatus, or device, or any suitable combination of the foregoing. More specific examples of a computer readable storage medium may include, but are not limited to, the following: an electrical connection having one or more wires, a portable computer diskette, a hard disk, a random access memory (RAM), a read-only memory (ROM), an erasable programmable read-only memory (EPROM or Flash memory), an optical fiber, a portable compact disc read-only memory (CD-ROM), an optical storage device, a magnetic storage device, or any suitable combination of the foregoing. In the context of the present disclosure, a computer readable storage medium may be any tangible medium that can contain, or store, a program for use by or in connection with an instruction execution system, apparatus, or device.

A computer readable signal medium may include a propagated data signal with computer readable program code embodied therein, for example, in baseband or as part of a carrier wave. Such a propagated signal may take any of a variety of forms, including, but not limited to, electro-magnetic, optical, or any suitable combination thereof. A computer readable signal medium may be any computer readable medium that is not a computer readable storage medium and that can communicate, propagate, or transport a program for use by or in connection with an instruction execution system, apparatus, or device.

Program code embodied on a computer readable medium may be transmitted using any appropriate medium, including but not limited to wireless, wireline, optical fiber, cable, RF, etc., or any suitable combination of the foregoing. Computer program code for carrying out operations for aspects of the present disclosure may be written in any combination of one or more programming languages, including an object oriented programming language such as Java(TM), Smalltalk, C++ or the like and conventional procedural programming languages, such as the "C" programming language or similar programming languages. The program code may execute entirely on the user's computer, partly on the user's computer, as a stand-alone software package, partly on the user's computer and partly on a remote computer, or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider).

Aspects of the present disclosure are described below with reference to flowchart illustrations and/or block diagrams of methods, apparatus (systems), and computer program products according to embodiments of the present disclosure. It will be understood that each block of the flowchart illustrations and/or block diagrams, and combinations of blocks in the flowchart illustrations and/or block diagrams, can be implemented by computer program instructions. These computer program instructions may be provided to a processor, in particular a microprocessor or a central processing unit (CPU), of a general purpose computer, special purpose computer, or other programmable data processing apparatus to produce a machine, such that the instructions, which execute via the processor of the computer, other programmable data processing apparatus, or other devices create means for implementing the functions/acts specified in the flowchart and/or block diagram block or blocks.

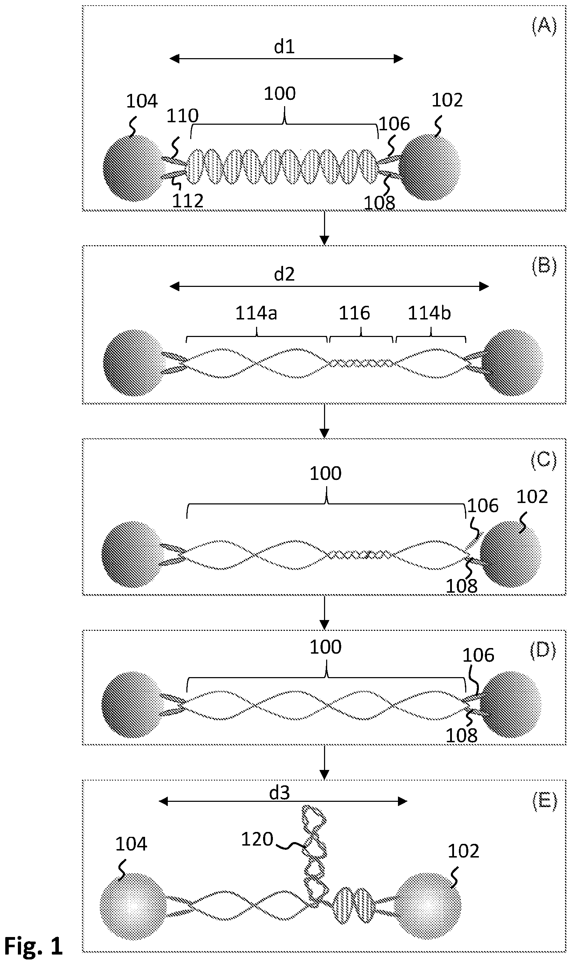

These computer program instructions may also be stored in a computer readable medium that can direct a computer, other programmable data processing apparatus, or other devices to function in a particular manner, such that the instructions stored in the computer readable medium produce an article of manufacture including instructions which implement the function/act specified in the flowchart and/or block diagram block or blocks, e.g. the blocks shown in FIG. 1.

The computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified in the flowchart and/or block diagram block or blocks.

The flowchart and block diagrams in the figures illustrate the architecture, functionality, and operation of possible implementations of systems, methods and computer program products according to various embodiments of the present disclosure. In this regard, each block in the flowchart or block diagrams may represent a module, segment, or portion of code, which comprises one or more executable instructions for implementing the specified logical function(s). It should also be noted that, in some alternative implementations, the functions noted in the blocks may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flowchart illustrations, and combinations of blocks in the block diagrams and/or flowchart illustrations, can be implemented by special purpose hardware-based systems that perform the specified functions or acts, or combinations of special purpose hardware and computer instructions.

In one aspect, embodiments of the present disclosure may relate to a computer-implemented method for supercoiling DNA.

In another aspect, embodiments of the present disclosure may relate to a computer-implemented method for determining a degree of supercoiling of the DNA molecule.

Moreover, a computer program for carrying out the methods described herein, as well as a non-transitory computer readable storage-medium storing the computer program are provided. A computer program may, for example, be downloaded (updated) to the existing system for supercoiling DNA (e.g. to the existing optical system and/or to the existing control module) or be stored upon manufacturing of these systems.

Elements and aspects discussed for or in relation with a particular embodiment may be suitably combined with elements and aspects of other embodiments, unless explicitly stated otherwise. Embodiments of the present disclosure will be further illustrated with reference to the attached drawings, which schematically will show embodiments according to the disclosure. It will be understood that the present disclosure is not in any way restricted to these specific embodiments.

## BRIEF DESCRIPTION OF THE DRAWINGS

Aspects of the disclosure will be explained in greater detail by reference to exemplary embodiments shown in the drawings, in which:

FIG. 1 illustrates method steps according to an embodiment,
FIG. 2 is a plot showing time versus degree of supercoiling of a DNA molecule illustrating the stability of a supercoiled state after performing steps according to an embodiment,
FIG. 3 is a plot from the literature showing the dependence of a streptavidin-biotin bond stability on the force applied to the bond,
FIG. 4A schematically shows a DNA molecule having an end-capped end,
FIG. 4B schematically shows a DNA molecule having four ends of the backbone strands of a DNA molecule separately connected to the first and second body,
FIG. 5A is a plot from the literature showing extension of DNA versus applied tension to DNA,
FIG. 5B is a plot showing for the data in FIG. 5A the degree of supercoiling versus extension of DNA at a particular force,
FIG. 6 is a plot showing force-extension curves obtained while executing an embodiment of the method five times,
FIG. 7 schematically shows a system for supercoiling DNA,
FIG. 8 is a plot showing the mobility of a protein on DNA for non-supercoiled DNA and for DNA that was supercoiled in accordance with an embodiment,
FIG. 9 illustrates a data processing system according to one embodiment for controlling a trapping system for supercoiling DNA.

## DETAILED DESCRIPTION OF THE DRAWINGS

FIG. 1 schematically illustrates a method for supercoiling DNA according to one embodiment. Block A shows an initial state of a configuration comprising a DNA molecule 100, a first body 102 and a second body 104. The DNA molecule 100 is at one side, in particular at a first end, connected to a first body 102, in particular to a first bead, e.g. an optically-trapped bead, and at another side, in particular at a second end, connected to a second body 104, in particular a second bead.

Alternatively (not shown), the DNA molecule is at one end connected to a bead and at its other end to a surface, such as an immovable functionalized surface. In this case, the surface constitutes the second body as described herein.

In the initial state, the DNA molecule is torsionally constrained and associated with a first linking number. The torsional constraint may be the result of both the first and the second end of the DNA molecule each being respectively connected to the first 102 and second 104 body by means of at least two bonds, 106, 108 and 110, 112 respectively. The DNA molecule 100 thus cannot wind or unwind assuming that the two bodies 102 and 104 do not rotate significantly on the timescale of the experiment.

Alternatively (not shown) the DNA molecule 100 may be torsionally constrained in the initial state, because, in addition to the first end being connected by at least two bonds to the first body, the second end is connected to the second body 104 by means of one bond around which the DNA molecule 100 cannot rotate.

Bond 106 may be a streptavidin-biotin bond. Bond 108 in principle can be any type of bond around which the DNA molecule 100 can rotate when torsionally unconstrained. Bond 108 may also be a streptavidin-biotin bond.

The bonds 110, 112 may be any type of bond. For the remainder, it will be assumed that all bonds 106, 108, 110 and 112 are streptavidin-biotin bonds.

In another embodiment, a plurality of Dig - Anti-Dig bonds as described in Nucleic Acids Research, 2013, Vol. 41, No. 19 e179 and as described in Nature Methods, Vol. 12, NO.1, January 2015, 47, may be used for connecting the DNA molecule 100 to the first and/or second body.

Blocks B, C and D illustrate the DNA molecule being in the intermediate state. Block B shows a step according to an embodiment of increasing the distance between the first and second body from a distance d1 to a distance d2.

The step of increasing this distance may comprise controlling the position of the first body relative to the second body using a trapping system, preferably an optical trapping system or an acoustical trapping system. The first and/or second body may namely be trapped in an optical or acoustical trap. With the increase of the distance, a torque is induced on the DNA molecule due to the intrinsic mechanical property of DNA that it wants to unwind as it is extended, e.g. overstretched. Furthermore, as increasing the distance increases a tension on the DNA molecule 100 and on the streptavidin-biotin bond 106, the bond 106 is destabilized. In addition, the induced torque may further destabilize the bond 106. As a result, the probability that bond 106 dissociates within a relatively short time period, e.g. 0.5 seconds to several minutes, is considerable.

The tension that is applied by increasing the distance between the beads preferably may be sufficient to induce at least part of the molecule to undergo the structural transition known as overstretching.

Preferably, the tension caused by the distance increase is higher than 80 pN, preferably higher than 100 pN, more preferably higher than 110 pN, most preferably higher than 115 pN. In torsionally constrained DNA, overstretching typically occurs at forces larger than approximately 115 pN. Due to the torsional constraint, the overall DNA linking number remains unchanged with respect to the initial situation. However, overstretching is permitted because some domains 114a and 114b of the DNA molecule underwind, while other domains 116 overwind. For a fully overstretched torsionally constrained DNA molecule, approximately 4/5 of the molecule's 100 length is underwound (with a helical twist of ~37.5 base-pairs per turn) and approximately 1/5 is overwound (with a helical twist of ~2.6 base-pairs per turn). In this way, the DNA molecule 100 can extend by up to ~70% without any change in overall linking number.

Preferably, after increasing the distance between the first and the second body from d1 to d2, the distance and/or tension is maintained substantially constant for a time period ranging between 0.5 seconds and several minutes.

Block C illustrates that at some point in time, the streptavidin-biotin bond 106 dissociates. As a result, the DNA molecule 100 is in a torsionally unconstrained state. After all, it is connected to the first body 102 by means of only one bond, namely bond 108. When bond 106 is dissociated, the induced torque can be at least partially released. The torque can in particular be released by the DNA molecule 100 swiveling around the single attachment point 108.

Block D illustrates that as a result of the torque release, the linking number of the DNA molecule has become lower with respect to the situations depicted in blocks A, B and C. Hence, the DNA molecule 100 is supercoiled. Block D further illustrates that the torsional constraint is only temporarily broken. The streptavidin-biotin bond 106 has reformed, herewith connecting the first body 102 and the DNA molecule 100. As a result, the DNA molecule 100 is again connected to the first body 102 by means of two bonds and the DNA molecule 100 is torsionally constrained again. Thus, by increasing the distance between the two bodies a torsionally constrained supercoiled DNA molecule 100 is obtained.

Block E illustrates the DNA molecule in the further state. As illustrated by block E, an embodiment of the method further comprises, after increasing said distance from d1 to d2, decreasing the distance between the first 102 and second 104 body for stabilizing a torsional constraint on the DNA molecule, in particular for stabilizing the at least one streptavidin-biotin bond 106. Herewith the changed linking number is preserved at lower forces. After the bond 106 has formed again, the distance between the two bodies, and the tension on the DNA, may be decreased before any of the bonds 106, 108 (or 110, 112) break again. Because the DNA molecule 100 is torsionally constrained, the helical twist is topologically forbidden to revert to its initial state shown in block A. Hence, the DNA molecule 100 does not revert to its initial non-supercoiled state. Instead, the DNA molecule 100 adopts a negatively-supercoiled state, wherein the linking number is lower than in the initial state, even at low forces, for example at forces below the forces associated with overstretching the DNA molecule. By the decrease of the distance, the tension on the DNA molecule may be reduced below 60 pN, preferably below 40 pN, more preferably below 20 pN, most preferably below 10 pN. This ensures the torsional constraint to be sufficiently stable.

As shown, decreasing the distance may result in the formation of at least one plectoneme 120. Advantageously, decreasing the distance and thereby decreasing the tension on the DNA molecule and on the streptavidin-biotin bond 106 stabilizes bond 106. Once at lower forces (e.g. << 100 pN), the biotin-streptavidin bonds - and thus the negatively-supercoiled state of the DNA molecule - are very stable over long periods of time, despite the effective torsional stress caused by the supercoiling. Hence, the DNA molecule 100 in block E is a stable, torsionally constrained negatively supercoiled DNA molecule.

Thus, once negatively supercoiled DNA has been prepared at high force through biotin-streptavidin bond rupture and subsequent re-formation, the resulting negatively supercoiled DNA remains torque-constrained, even at low forces (where it would rather be B-DNA). B-DNA is the structure that non-supercoiled DNA typically adopts under physiological conditions. It consists of the classic Watson-Crick structure wherein there is a spacing of around 0.34 nanometers per base-pair, and about 10.5 base-pairs per turn.

Typically, the supercoiled DNA state in block E is stable in the sense that the linking number changes less than 5% during a period of hours/days at tensions below 40 pN, at least when using sufficiently long DNA molecules and/or bodies of larger size. The degree of supercoiling decreases at a rate of around one helical turn per 30-40 seconds at low forces when using bodies in the form of optically-trapped beads of 4.5 micrometer diameter in water.

It should be appreciated that this method is stochastic in nature, because the time period during which the streptavidin-biotin bond is dissociated is stochastic and can therefore not be predicted precisely. Furthermore, it is believed (Biophysical Journal, Volume 89, December 2005, 4374-4381) that the biotin-streptavidin bond can sit in either of two energy minima, a deeper energy minimum and a shallower energy minimum. It could be that only bonds that sit within the deeper energy minimum are stable enough at low applied forces to stably torsionally constrain the DNA molecule. These effects may cause that embodiments described herein may yield stable supercoiled DNA at low forces for 30-40% of the times an embodiment of the method is performed, e.g. that are stable at forces below 100 pN for more than 30-40 minutes.

Preferably, in situation E depicted in FIG. 1, neither of the two bodies 102 and 104 significantly rotate as a result of the torque on the DNA molecule caused by the DNA molecule being in a supercoiled state. This is however easily ensured. To illustrate, a well-known formula for calculating the rotational velocity ($\Omega$) of an optically-trapped bead due to an applied torque ($\Gamma$) in a solution is:

$$\Omega = \Gamma / (8 * \pi \eta R) \qquad \text{(Eqn. 3)}$$

where $\eta$ is the solution viscosity and R is the bead diameter. For a solution with the viscosity of water, a bead of 4.5 micrometers, and a maximum torque of 10 pN nm, it would take ~30-40 seconds for a single bead rotation. The rotational speed is highly sensitive to the bead diameter, and thus significantly longer timescales can be obtained using even larger beads. Another factor is the length of the DNA molecule 100. Assuming a bead diameter of 4.5 micrometers, it will take more than 40 hours for a DNA molecule of ~48000 base-pairs (e.g. lambda phage DNA) to fully recoil. Within 30 minutes, only a small (< 2%) fraction of this molecule will have recoiled (assuming a torque of 10 pN nm). Hence, significant rotation of either one of the bodies is easily prevented.

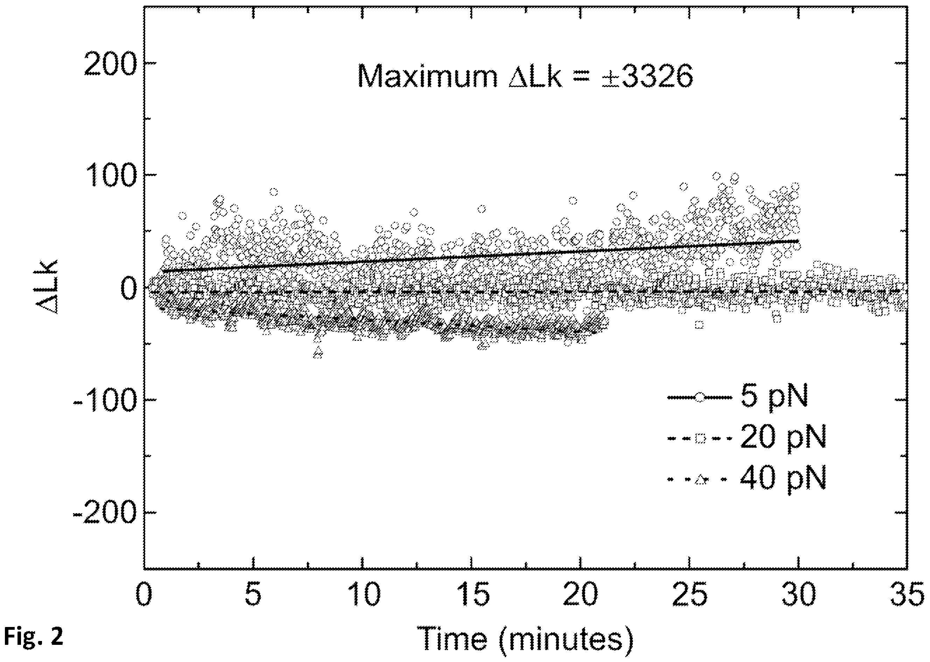

FIG. 2 is a plot showing how an average linking number Lk of three groups of approximately five supercoiled DNA molecules as per situation E of FIG. 1 changes with time. The plot particularly shows that the average linking number of each group, having an average sigma value of approximately -0.7, changes less than 5% over a period of 30-40 minutes when constant forces of 5, 20 and 40 pN respectively are applied to each group. For this plot, the difference in linking number was determined based on changes in extension of the DNA molecule, while a constant force of 5, 20 and 40 pN was applied to the molecules in each group respectively.

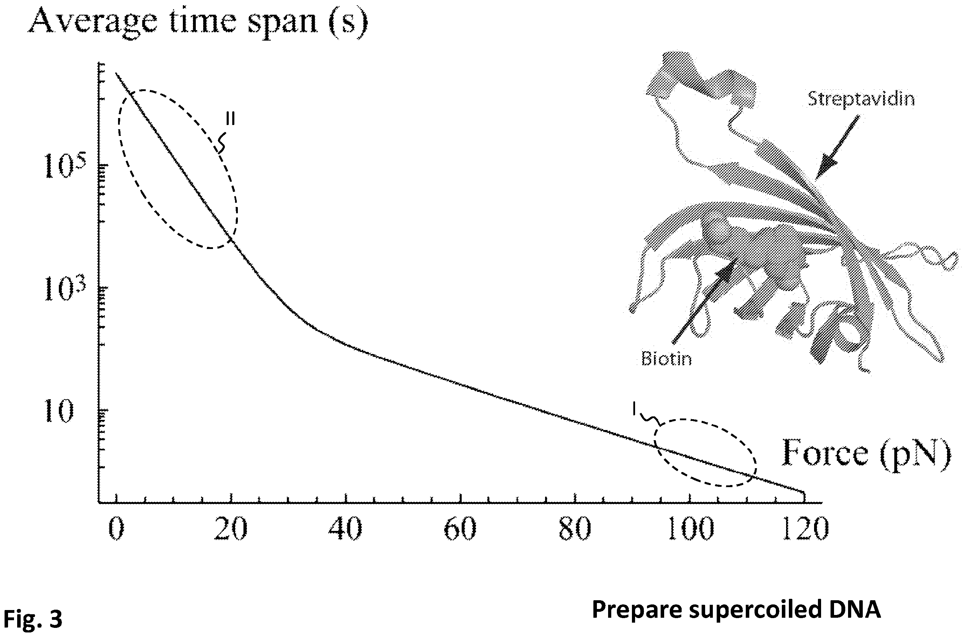

In one embodiment, an important cause for the stable linking number at low forces, in addition to limited rotation of the two bodies, is the high stability of the streptavidin-biotin bonds. FIG. 3 (Biophysical Journal, 89, 4374 (2005)) shows that at tensions of around 115 pN, the average time span of a streptavidin-biotin bond is predicted to be in the order of seconds (see region I), meaning that on average a streptavidin-biotin bond dissociates after a time period of the order of seconds. However, at low tensions, the average time span is orders of magnitude longer, e.g. hours (see region II).

Forming the configuration as per block A of FIG. 1 may comprise providing the first body comprising streptavidin for forming a bond with a biotin molecule, providing the at least part of the DNA molecule having one or more biotin molecules at its first end and optionally at its second end and combining the first body and the DNA molecule in a fluid for causing a bond to form between said streptavidin of the first body and the at least one biotin molecule bonded to the DNA molecule. The first body may comprise the streptavidin at its surface. The one or more biotin molecules may be covalently bonded to the DNA molecule. The first body and/or second body may be chemically attached to the streptavidin. In an example, the first body and/or the second body is at least partially coated with streptavidin.

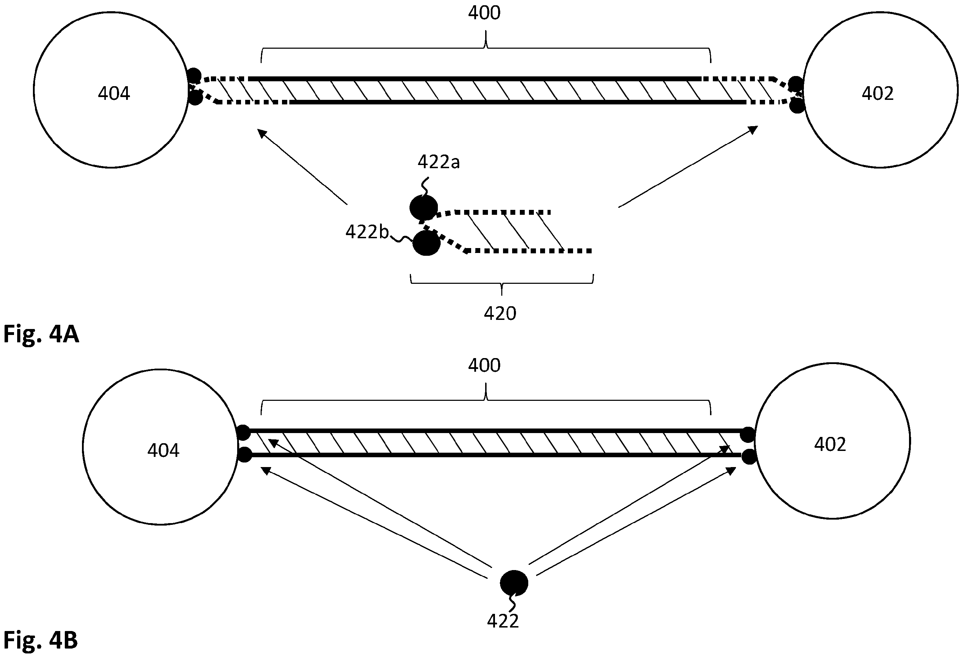

FIG. 4A shows that in one embodiment, forming a configuration as per block A in FIG. 1 comprises attaching an end-cap structure 420 to at least one end of the DNA molecule 400. As a result, the DNA molecule 400 has at least one looped end. If both ends of the DNA molecule 400 have an end-capped structure, the DNA molecule is topologically-closed, e.g. has no free and/or loose ends. Attaching the end-cap structure may be performed as described in King, G. A. et al. Unravelling the structural plasticity of stretched DNA under torsional constraint. Nat. Commun. 7:11810 doi: 10.1038/ncomms11810 (2016), in particular as described in the Supplementary Information section of this article. FIG. 4 particularly shows that an end-cap structure 420 is attached to both ends of the DNA molecule 400. For clarity, FIGs 4A and 4B do not show the helical twist of the two strands.

The end-cap structure 420 comprises at least one biotin molecule 422a, 422b for forming a bond with streptavidin. The end-cap structure 420 optionally comprises at least two, for example two, three or four, biotin molecules 422. The biotin molecules 422 may be chemically attached to the end-cap structure 420. Preferably, the at least one biotin molecule 422 is positioned at and/or near an end point of the loop structure 420. By attaching the end-cap structure 420 to the DNA molecule 400 the at least one biotin molecule 422 is connected to the DNA molecule.

In an embodiment, wherein the DNA molecule is connected to the first and second body only by means of streptavidin-biotin bonds, the stem loop structure comprises at least two biotin molecules, which can form a bond with the streptavidin that is present on the first and second bodies.

In a particular example, the end-cap structure 422 may comprise a 5T loop, which is a sequence of DNA that contains 5 'T' bases / nucleotides in a row. The 5T loop may be adjoined to a 12 base-pair double-stranded stem and a 12 nucleotide single-stranded overhang. The latter may be complementary to either the left or right cos site of lambda DNA, respectively.

Below are the examples of sequences for an end-cap that can be ligated to lambda DNA. The sequences between vertical dashes represent the four relevant domains of each oligomer, which from left to right are: the single-stranded overhang, one strand of the double-stranded stem, the 5T loop and the complementary strand of the double-stranded stem. The underlined nucleotides are labelled with a biotin.

End-cap example 1:

```
5' | AGG TCG CCG CCC | GGA GTT GAA CGT | TTTTT | ACG TTC
AAC TCC | 3'
```

End-cap example 2:

```
5' | GGG CGG CGA CCT | CAA GTT GGA CAA | TTTTT | TTG TCC AAC
TTG | 3'
```

End-capped torsionally constrained DNA may be prepared as follows. Lambda DNA and the two end-caps are first phosphorylated by reaction with polynucleotide kinase (NEB), as follows:

- Prepare phosphorylated lambda DNA (14 nM): lambda DNA (50 microliter, 250 micrograms/ml) + 10x T4 DNA ligase buffer (5.5 microliter) + polynucleotide kinase (0.5 microliter, 10,000 units/ml).
- Prepare phosphorylated end-cap as per example #1 (10 microM): end-cap #1 (2 microliter, 100 microM) + deionized water (15.5 microliter) + 10x T4 DNA ligase buffer (2 microliter) + polynucleotide kinase (0.5 microliter, 10,000 units/ml).
- Prepare phosphorylated end-cap as per example #2 (10 microM): end-cap #2 (2 microliter, 100 microM) + deionized water (15.5 microliter) + 10x T4 DNA ligase buffer (2 microliter) + polynucleotide kinase (0.5 microliter, 10,000 units/ml).

These reactions are left for one hour at 37°C. Following phosphorylation, end-cap #1 is incubated with lambda DNA in ~10-fold excess, as follows: 10x T4 DNA ligase buffer (50 microliter) + de-ionized water (400 microliter) + phosphorylated lambda DNA (56 microliter, 14 nM) + phosphorylated end-cap #1 (1 microliter, 10 microM).

To ensure that the end-cap anneals in its minimum energy configuration prior to the ligation, the above mixture may be heated to 80°C for five minutes, before being cooled rapidly on ice. Next, T4 DNA ligase enzyme (4 microliter, 200 units) is added, and the solution is left to react at 16°C for 12 hours.

Phosphorylated end-cap #2 (10 microliter, 10 microM) is then added to the above solution (in ~100-fold excess to lambda DNA). The mixture is heated to 80°C for five minutes, and cooled rapidly on ice. T4 DNA ligase enzyme (4 microliter, 200 units) is subsequently added to the solution and left to react at 16°C for 12 hours. Once completed, the ligated DNA construct may be purified and extracted, e.g. by ethanol precipitation, and may be stored in TE buffer.

Once each end-cap is ligated to lambda DNA, the DNA molecule may be tethered between two streptavidin-coated beads via biotin-streptavidin bonds. The number of biotins on end-cap #1 and end-cap #2 which bind to a given bead is variable. In the majority of cases (typically ~80%), at least two biotins on each end of the DNA molecule bind to a bead. This renders the DNA molecule unable to change its total linking number and the molecule is thus torsionally constrained.

Another option to provide torsionally constrained DNA 400 (as for example described in Van Mameren et al, PNAS, 106, 18231 (2009).) is to attach, e.g. chemically attach, at one end of the DNA molecule at least one biotin molecule 422 to a free backbone strand of the DNA molecule 400 for connecting the strand to the first body. One embodiment comprises binding at least one biotin molecule 422 to two free ends of the two respective strands at one end of the DNA molecule 400. This embodiment may also comprise binding biotin moieties 422 to all four free ends of the two strands of a DNA molecule 400. Then, if at least one biotin moiety of each strand binds to a streptavidin-coated surface, the DNA is torsionally constrained.

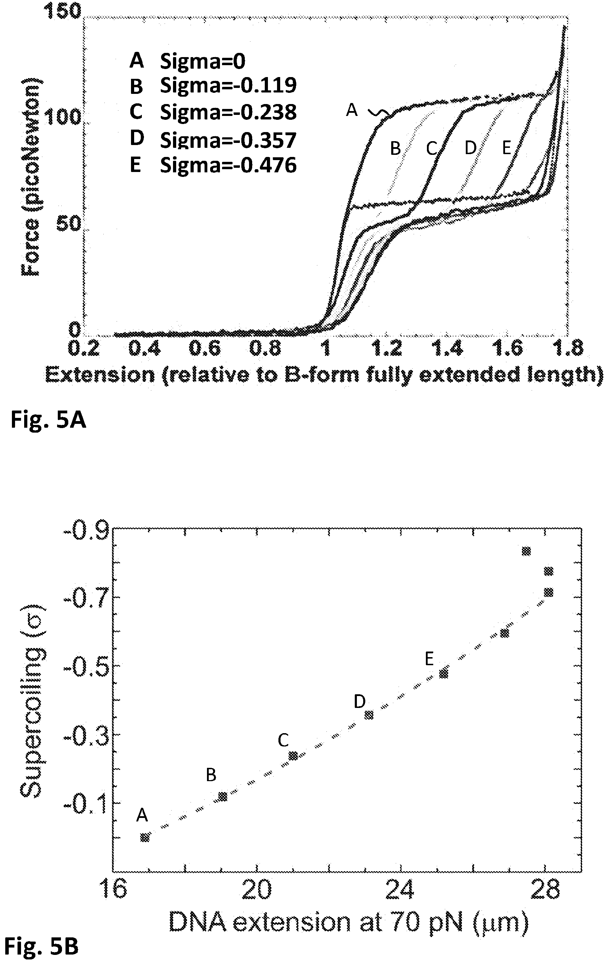

FIG. 5A shows reference information and in particular force-extension curves of supercoiled DNA as reported by Leger et al., Structural Transitions of a Twisted and Stretched DNA Molecule, Phys. Rev. Lett., 83, 1066 (1999). In these experiments the DNA was brought in a supercoiled state using micropipette tweezers. The DNA was held between an optical fiber and a bead (the latter trapped via suction in a micropipette). The optical fiber was used to stretch the DNA and the micropipette tweezers were used to rotate the DNA. The curves show that for increasing degrees of supercoiling, i.e. increasingly higher and negative sigma values, the extension of DNA at a particular force, e.g. at 70 pN, increases. FIG. 5B shows a plot of the degree of supercoiling against the extension of DNA under a tension of 70 pN. At 70 pN, a substantially linear relationship exists between DNA extension and the degree of negative supercoiling in the range of sigma between 0 and ~-0.7. So based on the extension of the DNA molecule at 70 pN the degree of supercoiling can be conveniently determined.

Thus by measuring an extension of a supercoiled DNA at a particular tension, e.g. 70 pN, the degree of supercoiling can be determined. Preferably, for determining the degree of supercoiling achieved using methods described herein, the conditions during the execution of the methods described herein are the same as the conditions that were present during the experiments in which the reference information was obtained. In particular, supercoiled DNA is positioned in a similar solution as was used when the reference information was obtained.

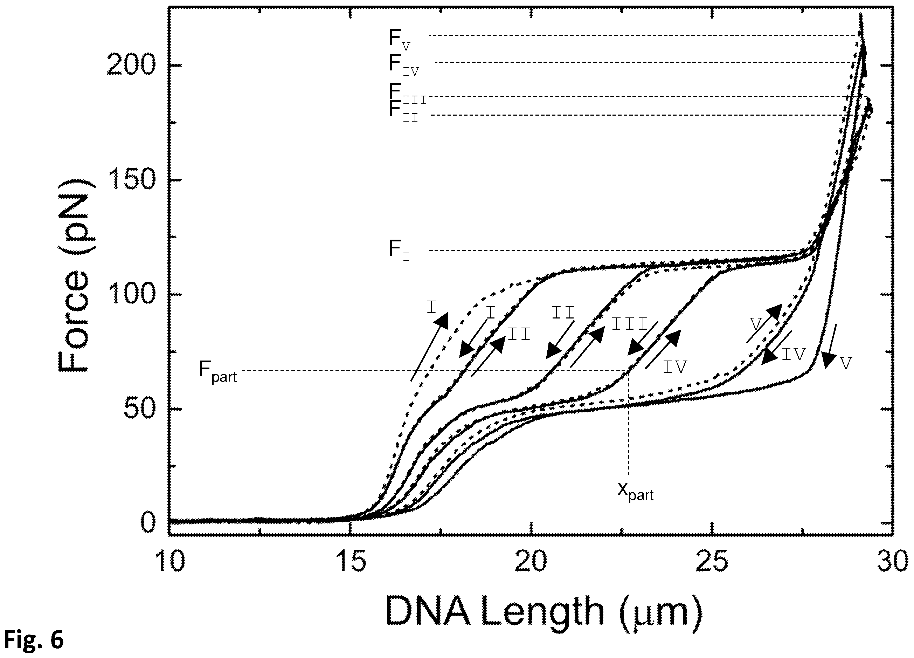

FIG. 6 shows force-extension curves that were actually measured during performing an embodiment of the method five times. In particular, a DNA molecule having a contour length of approximately 16 micrometers and fixed between two beads was extended and subsequently shortened five times. The dashed curves were measured during increasing the distance between the beads and the solid curves during decreasing the distance between the beads.

In the first experiment, denoted by I, the distance increase between the two beads caused a maximum tension in the DNA molecule of $F_I$. The force-extension curve associated with this distance increase is indicated by the upward arrow I. Subsequently, the distance between the two beads was decreased herewith releasing the tension of the molecule. The force-extension curve associated with this distance decrease is indicated by downward arrow I.

In the second experiment, denoted by II, the distance increase between the two beads caused a maximum tension in the DNA molecule of $F_{II}$. The force-extension curve associated with this distance increase is indicated by the upward arrow II, which coincides with the curve indicated by downward arrow I. Subsequently, the distance between the two beads was decreased again herewith releasing the tension of the molecule. The force-extension curve associated with this distance decrease is indicated by downward arrow II.

In the third experiment, denoted by III, the distance increase between the two beads caused a maximum tension in the DNA molecule of $F_{III}$. The force-extension curve associated with this distance increase is indicated by the upward arrow III, which coincides with the curve indicated by downward arrow II. Subsequently, the distance between the two beads was decreased again herewith releasing the tension of the molecule. The force-extension curve associated with this distance decrease is indicated by downward arrow III.

In the fourth experiment, denoted by IV, the distance increase between the two beads caused a maximum tension in the DNA molecule of $F_{IV}$. The force-extension curve associated with this distance increase is indicated by the upward arrow IV, which coincides with the curve indicated by downward arrow III. Subsequently, the distance between the two beads was decreased again herewith releasing the tension of the molecule. The force-extension curve associated with this distance decrease is indicated by downward arrow IV.

In the fifth experiment, denoted by V, the distance increase between the two beads caused a maximum tension in the DNA molecule of $F_V$. The force-extension curve associated with this distance increase is indicated by the upward arrow V, which coincides with the curve indicated by downward arrow IV. Subsequently, the distance between the two beads was decreased again herewith releasing the tension of the molecule. The force-extension curve associated with this distance decrease is indicated by downward arrow V.

As explained with reference to FIGs. 5A and 5B, based on a particular combination of tension $F_{part}$ and extension $x_{part}$, and based on reference information such as plotted in FIG. 5B, the degree of supercoiling can be determined. FIG. 6 shows that for increasing applied maximum tension to the DNA molecule, the extension of the DNA for a particular tension, e.g. $F_{part}$, which may be 70 pN, increases. Preferably, the particular tension is selected such that at this tension the dependence of the extension of the DNA molecule on the degree of supercoiling is relatively strong and/or linear. In particular, with the methods disclosed herein an average DNA linking number can be achieved that is between 0 % and ~70% lower than that of non-supercoiled (B-form) DNA, at forces $\leq$~115 pN.

In one embodiment, the method thus comprises obtaining reference information relating combinations of applied tension to DNA and extension of DNA to respective degrees of supercoiling of DNA and, based on a particular combination of tension on the DNA molecule and extension of the DNA molecule, e.g. occurring during decreasing the distance between the first and second body, and based on the reference information, determining a degree of supercoiling of the DNA molecule.

For increasing applied maximum tension to the DNA molecule, the degree of negative supercoiling thus increases on average. A possible explanation for this is that the amount of supercoiling generated depends on the length of time that the at least one biotin-streptavidin bond is broken for, which in turn depends on the stability of the bond. For instance, if the DNA molecule is free from torsional constraint (through breaking of a sufficient number of bonds) for >~1 second, the molecule can generally reduce its linking number as much as physically possible for a given induced torque. Conversely, if the torsional constraint is lost for only a fraction of this time, only a fraction of the maximum supercoiling will be generated. The amount of supercoiling generated can thus be tuned by controlling the stability of the bond.

Experimentally it was measured that for a sigma value of around -0.7, the DNA can rotate, when torsional constraint is removed, at a rate of around 4.5 rotations per millisecond. Hence it may take hundreds of milliseconds to fully 'release' all of the supercoiling.

Such tuning of the bond stability could be induced through other means (aside from force). This could include thermal, chemical or light induced breaking / reforming of the bonds. In one embodiment, the method comprises controlling at least one of a temperature of a fluid, a composition of a fluid and a light source for controlling a stability of the torsional constraint on the DNA molecule, in particular for controlling a stability of at least one streptavidin-biotin bond. The fluid may be understood to be the fluid in which the DNA molecule is positioned when the method is performed.

In one embodiment, the method comprises, based on a desired degree of supercoiling, determining a maximum force to be applied to the at least part of the DNA molecule and increasing the distance between the first and second body until the maximum force is applied to the DNA molecule. To this end, first, calibration information may be obtained that relates degrees of supercoiling to respective maximum forces applied to the DNA molecule. This calibration information may be obtained by performing a calibration experiment in accordance with the method for supercoiling as described herein, measuring the maximum applied force as a result of the distance increase and determining the degree of supercoiling that is achieved, e.g. based on the reference information as described above with reference to FIG. 5. Then, based on the thus obtained calibration information, the experiment may be repeated under the same conditions (on another, similar DNA molecule), wherein only the maximum applied force is changed to achieve the desired degree of supercoiling. This process may be iterated a number of times to accurately achieve the desired degree of supercoiling.

In one embodiment, the method comprises, based on a desired degree of supercoiling, determining a point in time to start decreasing the distance and decreasing the distance at said point in time. The point in time may be defined relative to the moment the maximum distance is reached, for example one second after the maximum distance is reached. In such case, the point in time effectively defines how long a maximum force is to be applied to the DNA molecule. It should be appreciated that the point in time may be determined based on calibration information that relates degrees of supercoiling to points in time to start decreasing the distance, for which calibration information may also be obtained by performing calibration experiments.

The calibration information preferably relates values for at least one of the following parameters to respective degrees of supercoiling:

- maximum applied force
- duration of maximum applied force
- solution conditions, such as temperature, pH, viscosity, composition
- speed with which the DNA molecule is extended
- type of bonds between the DNA molecule and the two bodies.

Of course, the calibration information may also relate combinations of at least two of such parameters to respective degrees of supercoiling.

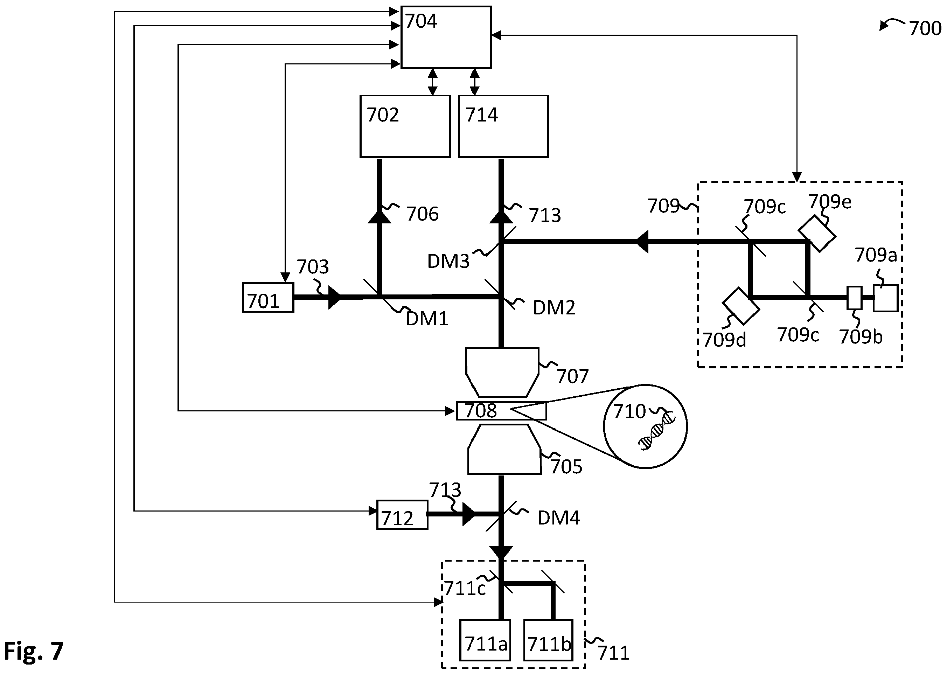

Fig. 6 demonstrates the principle that torsionally constrained supercoiled DNA can be generated, and then preserved at low force, e.g. below 15 pN, and amongst other things that the extent of supercoiling can be controlled by tuning the maximum applied force. The force-distance curves of FIG. 6 were measured whilst executing an embodiment of the method five times in a buffer of 20 mM tris-HCl, pH 7.6 with 25 mM sodium chloride. The measurements were performed using a dual-trap optical tweezers set-up, e.g. as shown in Fig. 7. Here, a non-supercoiled torsionally constrained DNA molecule was assembled *in situ* by tethering an end-capped linear DNA molecule as shown in Fig. 4A between two optically-trapped beads within a multi-channel microfluidic flow cell. The DNA (lambda phage DNA: 48502 base-pairs) was labelled with biotin moieties on each end-cap and had a contour length of ~16 micrometers. The beads were streptavidin-coated polystyrene particles of diameter 4.5 micrometers. The construction of the bead-DNA-bead assembly, as well as the design of the end-capped DNA molecule were as described in King et al., Nature Commun., 7, 11810 (2016). The data presented in Fig. 6 correspond to experiments in which the distance between the two beads (and thus the DNA extension) was increased and subsequently decreased sequentially through displacement of one of the beads with respect to the other. In each case, the DNA molecule was held at a maximum distance (corresponding to a maximum tension in the DNA) for 1 second prior to the distance being decreased (corresponding to the release of tension in the DNA). In the first of the five experiments (denoted by I), a non-supercoiled torsionally constrained DNA molecule was extended such that at least part of the molecule was overstretched. Here, a maximum tension in the DNA molecule of $F_I$ (~115 pN) was achieved at a distance of ~27 micrometers (~1.68 of its contour length). After 1 second, the distance between the two beads (and thus the tension in the DNA) was decreased; the hysteresis observed between the forward curve (upward arrow I) and backward curve (downward arrow I) indicate that the molecule become supercoiled at, or near, Fi. In the subsequent four experiments, the same DNA molecule was extended and then retracted, sequentially, so as to an induce increasingly high tension in the DNA molecule: ~180 pN ($F_{II}$), ~185 pN ($F_{III}$), ~195 pN ($F_{IV}$), and ~220 pN ($F_V$), respectively; on each occasion, the DNA was held at the maximum force ($F_{II}$, $F_{III}$, $F_{IV}$, or $F_V$) for 1 second before the distance was decreased. The velocity of the moving trap (and thus bead) for both increasing and decreasing the distance was approximately 0.3 micrometers per second.

FIG. 7 in particular illustrates a system 700 for supercoiling DNA according to one embodiment. The system 700 may be embodied as a microscope, for example at least one of a bright-field microscope, a confocal microscope, a fluorescence microscope, a Stimulated Emission Depletion (STED) microscope and a Total Internal Reflection (TIRF) microscope. The system 700 may comprise one or more light-sensitive systems 702, 714 and a control module 704. The light-sensitive systems 702, 714 may comprise a photo-detector and/or an imaging system, such as a camera, in particular a CCD camera such as an EMCCD camera and/or a position-dependent light sensor.

The system 700 may further comprise an excitation optical system comprising an excitation light source 701, such as an excitation laser, for example a 488, 532, 561 or 638 nm laser, in which the excitation optical system is configured to direct excitation light 703 towards the DNA molecule 710.

The light-sensitive system 702 may be configured to receive light 706 from the DNA molecule 710. To this end, the light-sensitive system 702 is for example positioned such that a light-sensitive part, such as an imaging plane, is directed towards the DNA molecule 710. The system 700 may comprise an objective lens 707 for collecting light 706 from the DNA molecule 710. Dichroic mirrors DM1 and DM2 may be used for separating the excitation light 703 and the detection light 706 and for coupling the excitation/detection path to the objective 707.

The DNA molecule 710 may be positioned in a sample holder 708. The optical system 700 may comprise such a sample holder in the form of a flow cell. The flow cell 708 may be a multichannel laminar flow cell that does not have a physical barrier between the channels enabling a fast buffer exchange between fluid flows respectively comprising beads, DNA, buffer and proteins. The sample holder 708 may comprise a piezo-controlled nano-stage for accurately positioning the sample.

The light-sensitive systems 702, 714 may be further configured to output a signal based on the received light 706, 713 respectively. The DNA molecule 710 for example is labeled with entities that are fluorescent and/or phosphorescent and/or luminescent and/or light absorbent, et cetera.

Preferably, the system 700 comprises a trapping system 709 for establishing a trap, e.g. an optical trap, that can hold the first and/or second body to which the DNA molecule 710 is connected. Optical traps are known in the prior art. An example of an optical trap is described in Neuman and Block, Rev. Sci. Instrum. 75, 2787 (2004). An example of an acoustical trap is disclosed in WO2014200341A1 with title "Molecular manipulation system and method".

In one embodiment the first and second body sit in respective traps, such as optical traps established by trapping system 709, and changing the distance between the first and second body comprises controlling the trapping system 709 to move said traps with respect to each other.

In one embodiment, the trapping system 709 comprises a trap light source 709a for generating trapping light. The trap light source 709a may be a 10 W 1064nm CW fiber laser. Further, trap system 709 may comprise a module 709b for modifying the polarization of the trapping light to facilitate splitting the trapping light into two light beams using a polarizing beam splitter 709c. One beam for establishing a first trap and a second for establishing a second trap. The trap system 709 may comprise a module 709d for controlling the position of the first trap and a module 709e for controlling the position of the second trap. In particular, independent trap steering may be done via accurate piezo mirrors 709d and 709e for the respective traps. If the trap system 709 is configured to establish two traps, advantageously the DNA molecule 710 can be held fixed between two optically trapped beads. The trapping beams are coupled into the objective using dichroic mirrors DM3 and DM2.

Preferably, the system 700 comprises a bright-field imaging system comprising an illumination source 712, such as an illumination light emitting diode, for example an 850 nm LED, in which the illumination source is configured to direct light 713 towards the sample via a dichroic mirror DM4 and a condenser 705. The bright-field imaging system further comprises a bright-field detection system 714 e.g. an imaging system, such as a camera, in particular a CCD camera and/or a CMOS camera for detection of illumination light 713 passing through the sample. The bright-field imaging system enables efficient detection of the trapped bodies, that may be optically trapped beads.

Preferably, the system 700 comprises a force detection system 711, that is configured to detect a force exerted by at least one of the traps established by trap system 709 on the DNA molecule. The system shown comprises a force detection module 711a for detecting a force exerted by the first trap and a force detection module 711b for detecting a force exerted by the second trap. As known in the art, these modules 711a and 711b may be position dependent sensors as the force can be determined based on a deflection of the trapping light and using back-focal plane interferometry. The light from the first and second traps may be separated by a polarizing beam splitter 711c.

The control module 704, as indicated by the double arrows, may be configured to control at least one of the light-sensitive system 702, the bright-field imaging system 714, the sample holder 708, the excitation optical system, in particular excitation light source 701, and the trapping system 709, in particular at least one of the trap light source 709a, module 709b, 709d and 709e.

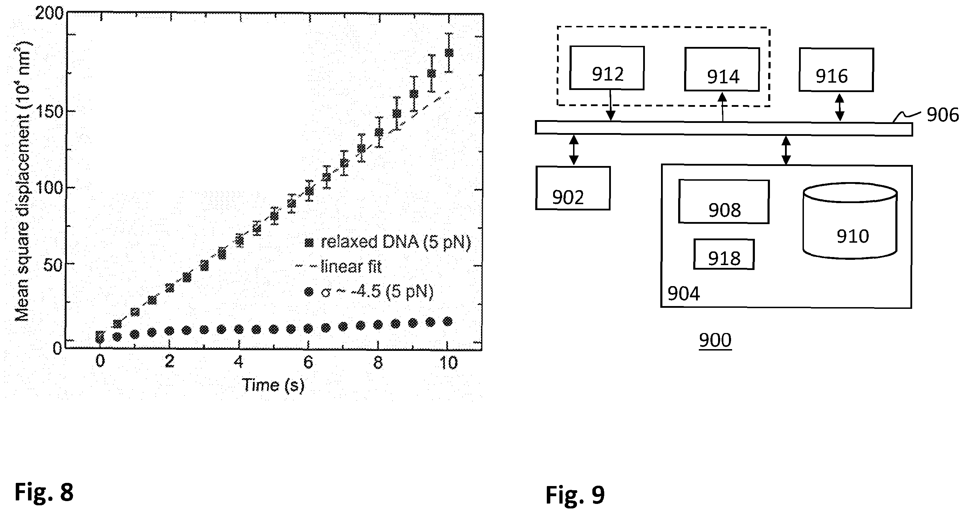

FIG. 8 shows how negative supercoiling can influence protein dynamics on DNA. FIG. 8 is a plot that shows the mobility of the mitochondrial protein TFAM (Mitochondrial Transcription Factor A) on DNA both for relaxed DNA and for DNA that was negatively supercoiled using the methods and systems as described herein. It was demonstrated that the protein's mobility on DNA is significantly hindered by local underwound structures associated with negatively supercoiled DNA (at 5 pN), resulting in highly confined diffusion. This finding has important implications for the ability of TFAM to search for its promoter sites in the mitochondrial genome and suggests that supercoiling could regulate this process. This illustrates that the methods disclosed herein provide a significant addition to the single-molecule toolkit, with potential applications ranging from fundamental studies of DNA and DNA-protein interactions, to research into pharmaceuticals and therapeutics.

Fig. 9 depicts a block diagram illustrating an exemplary data processing system that may be used in a computing system as described with reference to Fig. 2.

As shown in Fig. 9, the data processing system 900 may include at least one processor 902 coupled to memory elements 904 through a system bus 906. As such, the data processing system may store program code within memory elements 904. Further, the processor 902 may execute the program code accessed from the memory elements 904 via a system bus 906. In one aspect, the data processing system may be implemented as a computer that is suitable for storing and/or executing program code. It should be appreciated, however, that the data processing system 900 may be implemented in the form of any system including a processor and a memory that is capable of performing the functions described within this specification.

The memory elements 904 may include one or more physical memory devices such as, for example, local memory 908 and one or more bulk storage devices 910. The local memory may refer to random access memory or other non-persistent memory device(s) generally used during actual execution of the program code. A bulk storage device may be implemented as a hard drive or other persistent data storage device. The processing system 900 may also include one or more cache memories (not shown) that provide temporary storage of at least some program code in order to reduce the number of times program code must be retrieved from the bulk storage device 910 during execution.

Input/output (I/O) devices depicted as an input device 912 and an output device 914 optionally can be coupled to the data processing system. Examples of input devices may include, but are not limited to, a keyboard, a pointing device such as a mouse, or the like. Examples of output devices may include, but are not limited to, a monitor or a display, speakers, or the like. Input and/or output devices may be coupled to the data processing system either directly or through intervening I/O controllers.

In an embodiment, the input and the output devices may be implemented as a combined input/output device (illustrated in Fig. 9 with a dashed line surrounding the input device 912 and the output device 914). An example of such a combined device is a touch sensitive display, also sometimes referred to as a "touch screen display" or simply "touch screen". In such an embodiment, input to the device may be provided by a movement of a physical object, such as e.g. a stylus or a finger of a user, on or near the touch screen display.

A network adapter 916 may also be coupled to the data processing system to enable it to become coupled to other systems, computer systems, remote network devices, and/or remote storage devices through intervening private or public networks. The network adapter may comprise a data receiver for receiving data that is transmitted by said systems, devices and/or networks to the data processing system 900, and a data transmitter for transmitting data from the data processing system 900 to said systems, devices and/or networks. Modems, cable modems, and Ethernet cards are examples of different types of network adapter that may be used with the data processing system 900.

As pictured in Fig. 9, the memory elements 904 may store an application 918. In various embodiments, the application 918 may be stored in the local memory 908, the one or more bulk storage devices 910, or apart from the local memory and the bulk storage devices. It should be appreciated that the data processing system 900 may further execute an operating system (not shown in Fig. 9) that can facilitate execution of the application 918. The application 918, being implemented in the form of executable program code, can be executed by the data processing system 900, e.g., by the processor 902. Responsive to executing the application, the data processing system 900 may be configured to perform one or more operations or method steps described herein.

In one aspect of the present disclosure, the data processing system 900 may represent a control module as described herein.

Various embodiments of the disclosure may be implemented as a program product for use with a computer system, where the program(s) of the program product define functions of the embodiments (including the methods described herein). In one embodiment, the program(s) can be contained on a variety of non-transitory computer-readable storage media, where, as used herein, the expression "non-transitory computer readable storage media" comprises all computer-readable media, with the sole exception being a transitory, propagating signal. In another embodiment, the program(s) can be contained on a variety of transitory computer-readable storage media. Illustrative computer-readable storage media include, but are not limited to: (i) non-writable storage media (e.g., read-only memory devices within a computer such as CD-ROM disks readable by a CD-ROM drive, ROM chips or any type of solid-state non-volatile semiconductor memory) on which information is permanently stored; and (ii) writable storage media (e.g., flash memory, floppy disks within a diskette drive or hard-disk drive or any type of solid-state random-access semiconductor memory) on which alterable information is stored. The computer program may be run on the processor 902 described herein.

The terminology used herein is for the purpose of describing particular embodiments only and is not intended to be limiting of the disclosure. As used herein, the singular forms "a," "an," and "the" are intended to include the plural forms as well, unless the context clearly indicates otherwise. It will be further understood that the terms "comprises" and/or "comprising," when used in this specification, specify the presence of stated features, integers, steps, operations, elements, and/or components, but do not preclude the presence or addition of one or more other features, integers, steps, operations, elements, components, and/or groups thereof.

The corresponding structures, materials, acts, and equivalents of all means or step plus function elements in the claims below are intended to include any structure, material, or act for performing the function in combination with other claimed elements as specifically claimed. The description of embodiments of the present disclosure has been presented for purposes of illustration, but is not intended to be exhaustive or limited to the implementations in the form disclosed. Many modifications and variations will be apparent to those of ordinary skill in the art without departing from the scope of the present disclosure. The embodiments were chosen and described in order to best explain the principles and some practical applications of the present disclosure, and to enable others of ordinary skill in the art to understand the present disclosure for various embodiments with various modifications as are suited to the particular use contemplated.

## Claims

1.   A method for supercoiling DNA, wherein

in an initial state, at least part of a DNA molecule is torsionally constrained and associated with a first linking number and has a first end connected to a first body and a second end connected to a second body, the method comprising

increasing a distance between the first body and the second body for inducing a torque in the at least part of the DNA molecule and for bringing the at least part of the DNA molecule from the initial state into an intermediate state in which the at least part of the DNA molecule is temporarily torsionally unconstrained for at least partially releasing the induced torque for changing, e.g. decreasing, the first linking number, and

decreasing the distance between the first and second body for bringing the at least part of the DNA molecule from the intermediate state into a further state in which the at least part of the DNA molecule is torsionally constrained and associated with a second linking number different from the first linking number.

2.   The method according to claim 1, wherein the first end of the at least part of the DNA molecule is connected by at least two bonds to the first body when the at least part of the DNA molecule is torsionally constrained.

3.   The method according to claim 2, wherein the at least two bonds comprise a first bond and at least one other bond, the at least one other bond being a streptavidin-biotin bond.

4.   The method according to one or more of the preceding claims, wherein, when the at least part of the DNA molecule is torsionally constrained, the first end is connected by at least two streptavidin-biotin bonds to the first body and the second end is connected by at least two streptavidin-biotin bonds to the second body.

5.   The method according to any of claims 1-4 comprising controlling the position of the first body relative to the second body using a trapping system, preferably an optical trapping system or an acoustical trapping system.

6.   The method according to one or more of the preceding claims, wherein increasing the distance causes a tension in the DNA molecule higher than 80 pN, preferably higher than 100 pN, more preferably higher than 110 pN, most preferably higher than 115 pN.

7.   The method according to one or more of the preceding claims, wherein decreasing the distance reduces a tension in the DNA molecule below 100 pN, preferably below 80 pN, more preferably below 50 pN, most preferably below 20 pN or 10 pN.

8.   The method according to one or more of the preceding claims, comprising

providing the first body comprising streptavidin for forming a bond with a biotin molecule,
providing the at least part of the DNA molecule having a biotin molecule at its first end, and
combining the first body and the DNA molecule in a fluid for causing a bond to form between said streptavidin of the first body and the at least one biotin molecule bonded to the DNA molecule.

9. The method according to one or more of the preceding claims, wherein the DNA molecule comprises at its first and/or second end an end-cap structure.

10. The method according to one or more of the preceding claims, wherein, when the DNA molecule is torsionally constrained, two free backbone strands of the DNA molecule at the first end of the DNA molecule are each connected to the first body, and optionally
two free backbone strands of the DNA molecule at the second end of the DNA molecule are each connected to the second body.

11. The method according to one or more of the preceding claims, further comprising

obtaining reference information relating combinations of applied tension to DNA and extension of DNA to respective degrees of supercoiling of DNA, and
based on a particular combination of tension on the DNA molecule and extension of the DNA molecule, e.g. occurring during decreasing the distance between the first and second body, and based on the reference information, determining a degree of supercoiling of the DNA molecule.

12. The method according to one or more of the preceding claims, comprising,
based on a desired degree of supercoiling, determining a maximum force to be applied to the at least part of the DNA molecule and increasing the distance between the first and second body until the maximum force is applied to the DNA molecule.

13. The method according to one or more of the preceding claims, comprising,
based on a desired degree of supercoiling, determining a point in time to start decreasing the distance and decreasing the distance at said point in time.

14. The method according to one or more of the preceding claims, further comprising controlling at least one of a temperature of a fluid, a composition of a fluid and a light source for controlling a stability of the torsional constraint on the DNA molecule.

15. A system for supercoiling DNA, the system comprising

a trapping system for establishing at least one trap, e.g. an optical trap, for trapping at least one of a first and second body, wherein at least part of a DNA molecule has a first end connected to the first body and a second end connected to the second body, and
a control module for controlling the trapping system for controlling a position of the at least one trap, wherein the control module comprises a computer readable storage medium having computer readable program code embodied therewith, and a processor, preferably a microprocessor, coupled to the computer readable storage medium, wherein responsive to executing the computer readable program code, the processor is configured to perform executable operations comprising:

controlling the position of the at least one trap for increasing a distance between the first body and the second body for inducing a torque in the at least part of the DNA molecule and for bringing the at least part of the DNA molecule from the initial state into an intermediate state in which the at least part of the DNA molecule is temporarily torsionally unconstrained for at least partially releasing the induced torque for changing, e.g. decreasing, the first linking number, and
controlling the position of the at least one trap for decreasing the distance between the first and second body for bringing the at least part of the DNA molecule from the intermediate state into a further state in which the at least part of the DNA molecule is torsionally constrained and associated with a second linking number different from the first linking number.

**Patentansprüche**

1. Verfahren zum Supercoiling von DNA, wobei in einem Ausgangszustand zumindest ein Teil eines DNA-Moleküls torsionsbeschränkt ist und mit einer ersten Verknüpfungszahl (linking number) assoziiert ist und ein erstes Ende, das mit einem ersten Körper verbunden ist, und ein zweites Ende aufweist, das mit einem zweiten Körper verbunden ist, wobei das Verfahren umfasst: Vergrößern eines Abstands zwischen dem ersten Körper und dem zweiten Körper zum Induzieren eines Drehmoments (torque) in dem zumindest einen Teil des DNA-Moleküls und zum Versetzen des zumindest einen Teils des DNA-Moleküls vom Ausgangszustand in einen Intermediatszustand, in dem der zumindest eine Teil des DNA-Moleküls temporär nicht torsionsbeschränkt ist, um das induzierte Drehmoment zur Änderung, z.B. zum Herabsetzen, der ersten Verknüpfungszahl zumindest teilweise freizusetzen, und Verringern des Abstands zwischen dem ersten und dem zweiten Körper, zum Versetzen des zumindest einen Teils des DNA-Moleküls vom Intermediatszustand in einen weiteren Zustand, in dem der zumindest eine Teil des DNA-Moleküls torsionsbeschränkt ist und mit einer zweiten Verknüpfungszahl assoziiert ist, die sich von der ersten Verknüpfungszahl unterscheidet.

2. Verfahren nach Anspruch 1, wobei das erste Ende des zumindest einen Teils des DNA-Moleküls durch mindestens zwei Bindungen mit dem ersten Körper verbunden ist, wenn der zumindest eine Teil des DNA-Moleküls torsionsbeschränkt ist.

3. Verfahren nach Anspruch 2, wobei die mindestens zwei Bindungen eine erste Bindung und mindestens eine andere Bindung umfassen, wobei die mindestens eine andere Bindung eine Streptavidin-Biotin-Bindung ist.

4. Verfahren nach einem oder mehreren der vorangehenden Ansprüche, wobei, wenn der zumindest eine Teil des DNA-Moleküls torsionsbeschränkt ist, das erste Ende durch mindestens zwei Streptavidin-Biotin-Bindungen an den ersten Körper gebunden ist und das zweite Ende durch mindestens zwei Streptavidin-Biotin-Bindungen an den zweiten Körper gebunden ist.

5. Verfahren nach einem der Ansprüche 1 bis 4, umfassend das Steuern der Position des ersten Körpers im Verhältnis zu dem zweiten Körper unter Verwendung eines Trapping-Systems, bevorzugt eines optischen Trapping-Systems oder eines akustischen Trapping-Systems.

6. Verfahren nach einem oder mehreren der vorangehenden Ansprüche, wobei das Vergrößern des Abstands eine Spannung in dem DNA-Molekül verursacht, die höher ist als 80 pN, bevorzugt höher als 100 pN, stärker bevorzugt höher als 110 pN, am stärksten bevorzugt höher als 115 pN.

7. Verfahren nach einem oder mehreren der vorangehenden Ansprüche, wobei das Verringern des Abstands eine Spannung in dem DNA-Molekül auf unter 100 pN, bevorzugt unter 80 pN, stärker bevorzugt unter 50 pN, am stärksten bevorzugt unter 20 pN oder 10 pN verringert.

8. Verfahren nach einem oder mehreren der vorangehenden Ansprüche, umfassend:

Bereitstellen des ersten Körpers, der Streptavidin umfasst, zur Bildung einer Bindung mit einem Biotinmolekül, Bereitstellen des zumindest einen Teils des DNA-Moleküls, das ein Biotinmolekül an seinem ersten Ende aufweist, und Kombinieren des ersten Körpers und des DNA-Moleküls in einer Flüssigkeit, so dass sich eine Bindung zwischen dem Streptavidin des ersten Körpers und dem mindestens einen an das DNA-Molekül gebundene Biotinmolekül bildet.

9. Verfahren nach einem oder mehreren der vorangehenden Ansprüche, wobei das DNA-Molekül an seinem ersten und/oder zweiten Ende eine End-Cap-Struktur umfasst.

10. Verfahren nach einem oder mehreren der vorangehenden Ansprüche, wobei, wenn das DNA-Molekül torsionsbeschränkt ist, zwei freie Rückgradstränge des DNA-Moleküls am ersten Ende des DNA-Moleküls jeweils mit dem ersten Körper verbunden sind, und gegebenenfalls zwei freie Rückgradstränge des DNA-Moleküls am zweiten Ende des DNA-Moleküls jeweils mit dem zweiten Körper verbunden sind.

11. Verfahren nach einem oder mehreren der vorangehenden Ansprüche, des Weiteren umfassend:

Erhalten von Referenzinformation, die Kombinationen von angewandter Spannung auf DNA und die Extension von DNA auf jeweilige Grade des Supercoilings von DNA bezieht, und auf Grundlage einer bestimmten Kombination von Spannung auf dem DNA-Molekül und Extensionen des DNA-Moleküls, die z.B. während des Verringerns des Abstandes zwischen dem ersten und zweiten Körper auftreten, und auf Grundlage der Referenzinformation, Bestimmen eines Grades an Supercoiling des DNA-Moleküls.

12. Verfahren nach einem oder mehreren der vorangehenden Ansprüche, umfassend:
auf Grundlage eines erwünschten Grades an Supercoiling, Bestimmen einer maximalen Kraft, die auf den zumindest einen Teil des DNA-Moleküls ausgeübt wird, und Vergrößern des Abstands zwischen dem ersten und zweiten Körper, bis die maximale Kraft auf das DNA-Molekül ausgeübt wird.

13. Verfahren nach einem oder mehreren der vorangehenden Ansprüche, umfassend:
auf Grundlage eines gewünschten Grades an Supercoiling, Bestimmen eines Zeitpunkts zum Beginnen des Verringerns des Abstands und Verringern des Abstands zu diesem Zeitpunkt.

14. Verfahren nach einem oder mehreren der vorangehenden Ansprüche, des Weiteren umfassend das Steuern von mindestens einem, einer Temperatur einer Flüssigkeit, einer Zusammensetzung einer Flüssigkeit und einer Lichtquelle zum Steuern einer Stabilität der Torsionsbeschränkung des DNA-Moleküls.

15. System zum Supercoiling von DNA, wobei das System umfasst:

ein Trapping-System zum Errichten mindestens einer Trap, z.B. einer optischen Trap, zum Trapping mindestens eines aus einem ersten und zweiten Körper, wobei zumindest ein Teil eines DNA-Moleküls ein erstes Ende, das mit dem ersten Körper verbunden ist, und ein zweites Ende, das mit dem zweiten Körper verbunden ist, aufweist, und
ein Steuermodul zum Steuern des Trapping-Systems zum Steuern einer Position der mindestens einen Trap, wobei
das Steuermodul ein computerlesbares Speichermedium, das einen computerlesbaren Programmcode aufweist, der darin gespeichert ist, und einen Prozessor, bevorzugt einen Microprozessor, der an das computerlesbare Speichermedium gekoppelt ist, umfasst, wobei der Prozessor als Reaktion auf das Ausführen des computerlesbaren Programmcodes konfiguriert ist, um ausführbare Abläufe durchzuführen, umfassend:

Steuern der Position der mindestens einen Trap zum Vergrößern eines Abstands zwischen dem ersten Körper und dem zweiten Körper zum Induzieren eines Drehmoments in dem zumindest einen Teil des DNA-Moleküls und zum Versetzen des zumindest einen Teils des DNA-Moleküls vom Ausgangszustand in einen Intermediatszustand, in dem der zumindest eine Teil des DNA-Moleküls temporär nicht torsionsbeschränkt ist, um das induzierte Drehmoment zur Änderung, z.B. zum Herabsetzen, der ersten Verknüpfungszahl zumindest teilweise freizusetzen, und
Steuern der Position der mindestens einen Trap zum Verringern des Abstands zwischen dem ersten und zweiten Körper zum Versetzen des zumindest einen Teils des DNA-Moleküls vom Intermediatszustand in einen weiteren Zustand, in dem der zumindest eine Teil des DNA-Moleküls torsionsbeschränkt ist und mit einer zweiten Verknüpfungszahl assoziiert ist, die sich von der ersten Verknüpfungszahl unterscheidet.

**Revendications**

1. Procédé de superenroulement d'ADN, dans lequel

dans un état initial, au moins une partie d'une molécule d'ADN est contrainte en torsion et associée à un premier nombre de liaisons et présente une première extrémité raccordée à un premier corps et une seconde extrémité raccordée à un second corps, le procédé comprenant
l'augmentation d'une distance entre le premier corps et le second corps pour induire un couple dans l'au moins une partie de la molécule d'ADN et pour faire passer l'au moins une partie de la molécule d'ADN de l'état initial dans un état intermédiaire dans lequel l'au moins une partie de la molécule d'ADN est temporairement non contrainte en torsion pour au moins partiellement libérer le couple induit pour changer, par exemple réduire, le premier nombre de liaisons, et
la réduction de la distance entre le premier et le second corps pour faire passer l'au moins une partie de la molécule d'ADN de l'état intermédiaire dans un autre état dans lequel l'au moins une partie de la molécule d'ADN est contrainte en torsion et associée à un second nombre de liaisons différent du premier nombre de liaisons.

**2.** Procédé selon la revendication 1, dans lequel la première extrémité de l'au moins une partie de la molécule d'ADN est raccordée par au moins deux liaisons au premier corps lorsque l'au moins une partie de la molécule d'ADN est contrainte en torsion.

**3.** Procédé selon la revendication 2, dans lequel les aux moins deux liaisons comprennent une première liaison et au moins une autre liaison, l'au moins une autre liaison étant une liaison streptavidine-biotine.

**4.** Procédé selon une ou plusieurs des revendications précédentes, dans lequel, lorsque l'au moins une partie de la molécule d'ADN est contrainte en torsion, la première extrémité est raccordée par au moins deux liaisons streptavidine-biotine au premier corps et la seconde extrémité est raccordée par au moins deux liaisons streptavidine-biotine au second corps.

**5.** Procédé selon l'une quelconque des revendications 1 à 4 comprenant la commande de la position du premier corps par rapport au second corps en utilisant un système de piégeage, de préférence un système de piégeage optique ou un système de piégeage acoustique.

**6.** Procédé selon une ou plusieurs des revendications précédentes, dans lequel l'augmentation de la distance entraîne une tension dans la molécule d'ADN supérieure à 80 pN, de préférence supérieure à 100 pN, de manière davantage préférée supérieure à 110 pN, de manière préférée entre toutes supérieures à 115 pN.

**7.** Procédé selon une ou plusieurs des revendications précédentes, dans lequel la réduction de la distance réduit une tension dans la molécule d'ADN au-dessous de 100 pN, de préférence au-dessous de 80 pN, de manière davantage préférée au-dessous de 50 pN, de manière préférée entre toutes au-dessous de 20 pN ou de 10 pN.

**8.** Procédé selon une ou plusieurs des revendications précédentes, comprenant

la fourniture du premier corps comprenant de la streptavidine pour former une liaison avec une molécule de biotine,
la fourniture de l'au moins une partie de la molécule d'ADN ayant une molécule de biotine au niveau de sa première extrémité, et
la combinaison du premier corps et de la molécule d'ADN dans un fluide pour entraîner la formation d'une liaison entre ladite streptavidine du premier corps et l'au moins une molécule de biotine liée à la molécule d'ADN.

**9.** Procédé selon une ou plusieurs des revendications précédentes, dans lequel la molécule d'ADN comprend au niveau de sa première extrémité et/ou de sa seconde extrémité une structure de coiffe terminale.

**10.** Procédé selon une ou plusieurs des revendications précédentes, dans lequel, lorsque la molécule d'ADN est contrainte en torsion, deux brins de squelette libres de la molécule d'ADN au niveau de la première extrémité de la molécule d'ADN sont chacun raccordés au premier corps, et facultativement,
deux brins de squelette libres de la molécule d'ADN au niveau de la seconde extrémité de la molécule d'ADN sont chacun raccordés au second corps.

**11.** Procédé selon une ou plusieurs des revendications précédentes, comprenant en outre

l'obtention d'informations de référence concernant des combinaisons de tension appliquée à l'ADN et d'extension d'ADN à des degrés respectifs de superenroulement d'ADN, et
sur la base d'une combinaison particulière de tension sur la molécule d'ADN et d'extension de la molécule d'ADN, par exemple survenant durant la réduction de la distance entre le premier et le second corps, et sur la base des informations de référence, la détermination d'un degré de superenroulement de la molécule d'ADN.

**12.** Procédé selon une ou plusieurs des revendications précédentes, comprenant,
sur la base d'un degré souhaité de superenroulement, la détermination d'une force maximale à appliquer à l'au moins une partie de la molécule d'ADN et l'augmentation de la distance entre les premier et second corps jusqu'à ce que la force maximale soit appliquée à la molécule d'ADN.

**13.** Procédé selon une ou plusieurs des revendications précédentes, comprenant,
sur la base d'un degré souhaité de superenroulement, la détermination d'un point dans le temps pour commencer la réduction de la distance et la réduction de la distance audit point dans le temps.

**14.** Procédé selon une ou plusieurs des revendications précédentes, comprenant en outre la commande d'au moins l'une d'une température de fluide, d'une composition d'un fluide et d'une source de lumière pour commander une stabilité de la contrainte en torsion sur la molécule d'ADN.

**15.** Système de superenroulement d'ADN, le système comprenant

un système de piégeage pour établir au moins un piège, par exemple un piège optique, pour piéger au moins l'un d'un premier et d'un second corps, dans lequel au moins une partie d'une molécule d'ADN a une première extrémité raccordée au premier corps et une seconde extrémité raccordée au second corps, et
un module de commande pour commander le système de piégeage pour commander une position de l'au moins un piège, dans lequel
le module de commande comprend un support de stockage lisible sur ordinateur ayant un code de programme lisible sur ordinateur mise en œuvre avec celui-ci, et un processeur, de préférence un microprocesseur, couplé au support de stockage lisible sur ordinateur, dans lequel en réponse à l'exécution du code de programme lisible sur ordinateur, le processeur est configuré pour réaliser des opérations exécutables comprenant :

la commande de la position de l'au moins un piège pour augmenter une distance entre le premier corps et le second corps pour induire un couple dans l'au moins une partie de la molécule d'ADN et pour faire passer l'au moins une partie de la molécule d'ADN de son état initial dans un état intermédiaire dans lequel l'au moins une partie de la molécule d'ADN est temporairement non contrainte en torsion pour au moins partiellement libérer le couple induit pour le changement, par exemple la réduction, du premier nombre de liaisons, et
la commande de la position de l'au moins un piège pour réduire la distance entre le premier et le second corps pour faire passer l'au moins une partie de la molécule d'ADN de l'état intermédiaire dans un autre état dans lequel l'au moins une partie de la molécule d'ADN est contrainte en torsion et associée à un second nombre de liaisons différent du premier nombre de liaisons.

**Fig. 1**

**Fig. 2**

EP 3 737 756 B1

Fig. 3

**Prepare supercoiled DNA**

Fig. 4A

Fig. 4B

**Fig. 5A**

**Fig. 5B**

Fig. 6

Fig. 7

**Fig. 8**

**Fig. 9**

EP 3 737 756 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2014200341 A1

**Non-patent literature cited in the description**

- **VAN LOENHOUT.** *M.T.J. Science,* 2012, vol. 338, 94
- *Colloids Surf B Biointerfaces,* 08 March 2011, vol. 3 (1), 91-95
- *Nucleic Acids Research,* 2013, vol. 41 (19), e179
- *Nature Methods,* January 2015, vol. 12 (1), 47
- *Biophysical Journal,* December 2005, vol. 89, 4374-4381
- *Biophysical Journal,* 2005, vol. 89, 4374
- **KING, G. A. et al.** Unravelling the structural plasticity of stretched DNA under torsional constraint. *Nat. Commun.,* 2016, vol. 7, 11810
- **VAN MAMEREN et al.** *PNAS,* 2009, vol. 106, 18231
- **LEGER et al.** Structural Transitions of a Twisted and Stretched DNA Molecule. *Phys. Rev. Lett.,* 1999, vol. 83, 1066
- **KING et al.** *Nature Commun.,* 2016, vol. 7, 11810
- **NEUMAN ; BLOCK.** *Rev. Sci. Instrum.,* 2004, vol. 75, 2787